# EUROPEAN PATENT APPLICATION

(11) **EP 4 837 904 A1**
(43) Date of publication of application: **11.11.2026**
(21) Application number: 24915180.4
(22) Date of filing: 26.12.2024
(51) Int. Cl.: C07D 401/12, C07D 405/14, C07D 405/12, A61K 31/444, A61K 45/06, A61P 1/00, A61P 35/00, A61P 31/04

(54) **PYRROLE SULFONAMIDE ANTACID CRYSTAL AND PREPARATION METHOD THEREFOR AND USE THEREOF, AND PHARMACEUTICAL COMPOSITION**

(30) Priority: 05.01.2024 CN 202410018458
(71) Applicant: Zhejiang Medicine Co., Ltd Xinchang Pharmaceutical Factory, Shaoxing, Zhejiang 312599 (CN); Zhejiang Changhai Pharmaceutical Co., Ltd., Shaoxing, Zhejiang 312366 (CN)
(72) Inventor: LI, Nanxing, Shaoxing, Zhejiang 312366 (CN); SHENG, Li, Shaoxing, Zhejiang 312366 (CN); FAN, Gang, Shaoxing, Zhejiang 312366 (CN); GAO, Haoling, Shaoxing, Zhejiang 312366 (CN); LIU, Jianhong, Shaoxing, Zhejiang 312366 (CN); TIAN, Lijie, Shaoxing, Zhejiang 312366 (CN); CHEN, Jing, Shaoxing, Zhejiang 312366 (CN); YUAN, Xiying, Shaoxing, Zhejiang 312366 (CN); ZHAO, Lin, Shaoxing, Zhejiang 312366 (CN); ZHAO, Zhongyuan, Shaoxing, Zhejiang 312366 (CN); WANG, Li, Shaoxing, Zhejiang 312366 (CN); WANG, Ping, Shaoxing, Zhejiang 312366 (CN)
(74) Representative: Barker Brettell LLP
(86) International application number: PCT/CN2024/142891
(87) International publication number: WO 2025/145965

(57) **Abstract**

Provided is pyrrole sulfonamide acid inhibitor crystal and preparation method and application thereof, and drug composition. Specifically, disclosed in the present disclosure is a pyrrole sulfonamide acid inhibitor crystal of a compound of a Formula I, and a chemical structural formula of the crystal is shown in the Formula I: 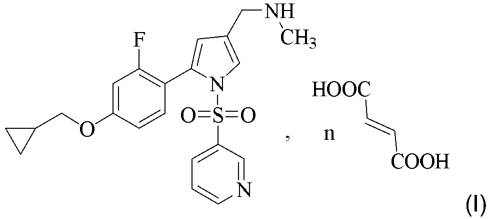 wherein, n=0.1-2.0. The crystal form is good in stability, fluidity, and humidity resistance, and the preparation method for the crystal form is simple in process, easy to control, and suitable for mass production.

## Description

This application claims priority to Chinese Patent Application No. 202410018458.3 filed to the China National Intellectual Property Administration on January 05, 2024, the disclosure of which is hereby incorporated by reference in its entirety.

### Technical Field

The present disclosure relates to the field of medicinal chemistry, and in particular, to pyrrole sulfonamide acid inhibitor crystal and preparation method and application thereof, and drug composition.

### Background

Acid-Related Diseases (ARDs) are a category of diseases that are triggered or caused by stomach acid attacks, and mainly include Peptic Ulcer Disease (PUD), Gastroesophageal Reflux Disease (GERD), etc. Among drug treatments for such diseases, acid-inhibiting drugs are clinically the most effective means of treating the Acid-Related Diseases. Although Proton Pump Inhibitors (PPIs) have occupied a dominant share in the field of acid inhibition, there are problems such as slow effect taking, unstable acid inhibition, large individual differences in efficacy, multiple drug interactions, etc.

Potassium Competitive Acid Blockers (P-CABs) bind non-covalently (hydrogen bonds and ionic bonds) to H⁺/K⁺-ATPase with K⁺ competitively, so as to inhibit the activity of the H⁺/K⁺-ATPase, and thus have strong and long-lasting inhibition of gastric acid secretion. 1-[5-[4-(cyclopropylmethoxy)-2-fluorophenyl]-1-(pyridin-3-ylsulfonyl)-1H-pyrrole-3-yl]-N-methylmeth anamine (CN 113620930 B) is used as the Potassium Competitive Acid Blockers. The compound shows an excellent inhibitory effect on gastric acid secretion and features of reducing hepatotoxicity, etc.

In an organic compound, many substances often have the same chemical composition and can form different crystal structures at different conditions such as temperatures, solvents, etc. Different polymorphs of a substance have different lattice energies, and thus show different chemical and physical properties in a solid state, including chemical stability, solubility, dissolution rates, etc. These properties may have a direct impact on the treatment and production of active pharmaceutical ingredients and preparations and affect the stability, solubility, and bioavailability of the active pharmaceutical ingredients and preparations. For the purposes of the present disclosure, there exists a need in the art: to obtain a polycrystalline form with excellent physicochemical properties suitable for application.

### Summary

The present disclosure is intended to provide a crystal form of a compound of a Formula I with good stability and a low water content.

The present disclosure is further intended to provide a method for preparing a crystal form of a compound of a Formula I.

A first aspect of the present disclosure provides a pyrrole sulfonamide acid inhibitor crystal of a compound of a Formula I.

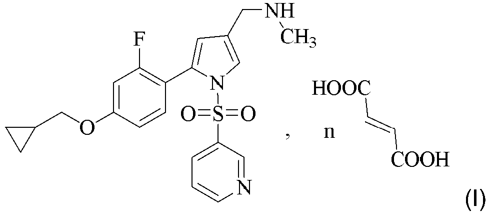

A crystal form of the crystal is selected from the following groups: a crystal form Form A, a crystal form Form B, a crystal form Form C, or a crystal form Form D, where n=0.1-2.0.

In another preferred embodiment, the crystal is an anhydrate.

In another preferred embodiment, n=0.1-2.0, preferably 0.5-1.5, and more preferably 0.5, 1, or 1.5.

In another preferred embodiment, n=0.5, 1.0, or 1.5.

In another preferred embodiment, an X-ray Powder Diffraction (XRPD) diagram of the crystal form Form A includes 3 or more than 3 (4, 5, or 6) 2θ values selected from the following groups: 4.1°±0.2°, 8.3°±0.2°, 12.5°±0.2°, 19.6°±0.2°, 20.9°±0.2°, 23.1 °±0.2°.

In another preferred embodiment, the XRPD diagram of the crystal form Form A includes 1 or more than 1 2θ values selected from the following groups: 4.1°±0.2°, 8.3°±0.2°, 12.5°±0.2°, 20.9°±0.2°, and 23.1°±0.2°.

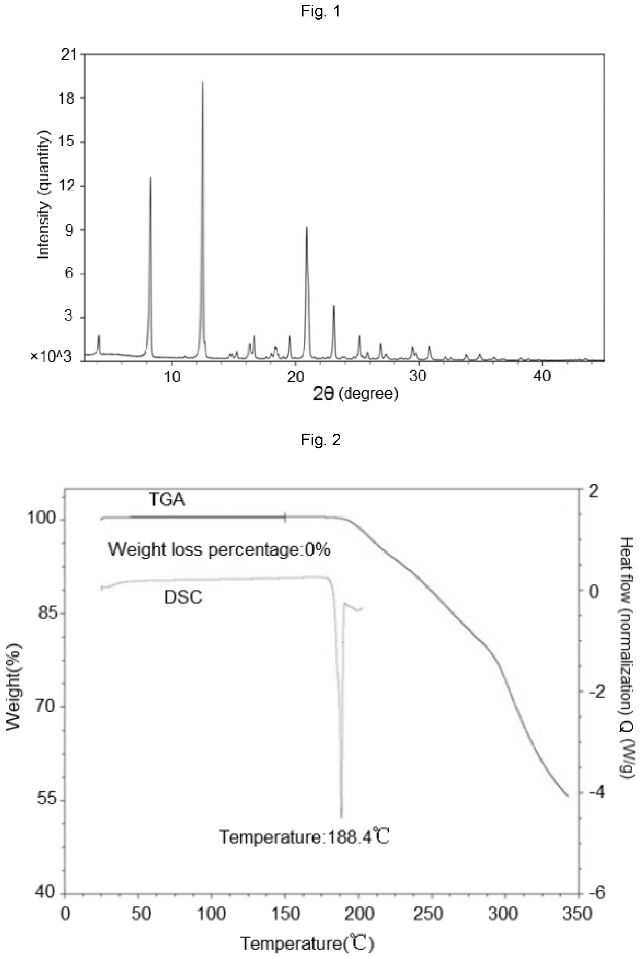

In another preferred embodiment, the crystal form Form A also has one or more features selected from the following groups.
(i1) The XRPD diagram of the crystal form Form A includes 6 or more than 6 2θ values selected from the following groups: 4.1°±0.2°, 8.3°±0.2°, 12.5°±0.2°, 16.7°±0.2°, 19.6°±0.2°, 20.9°±0.2°, 23.1°±0.2°, 25.2°±0.2°, and 26.9°±0.2°.
(i2) The XRPD diagram of the crystal form Form A is characterized in Fig. 1.
(i3) The crystal form Form A loses no weight at 20-150 °C.
(i4) A Thermogravimetric Analysis (TGA) diagram of the crystal form Form A is characterized in Fig. 2.
(i5) A peak temperature of a Differential Scanning Calorimetry (DSC) diagram of the crystal form Form A is 188.4 °C.
(i6) The DSC diagram of the crystal form Form A is characterized in Fig. 2.

In another preferred embodiment, n in the crystal form Form A is =1.

In another preferred embodiment, an X-ray Powder Diffraction (XRPD) diagram of the crystal form Form B includes 3 or more than 3 (4, 5, or 6) 2θ values selected from the following groups: 5.3°±0.2°, 7.5°±0.2°, 9.6°±0.2°, 14.4°±0.2°, 16.0°±0.2°, 18.7°±0.2°, and 22.1°±0.2°.

In another preferred embodiment, the XRPD diagram of the crystal form Form B includes 1 or more than 1 2θ values selected from the following groups: 5.3°±0.2°, 9.6°±0.2°, 14.4°±0.2°, 16.0°±0.2°, 18.7°±0.2°, and 22.1°±0.2°.

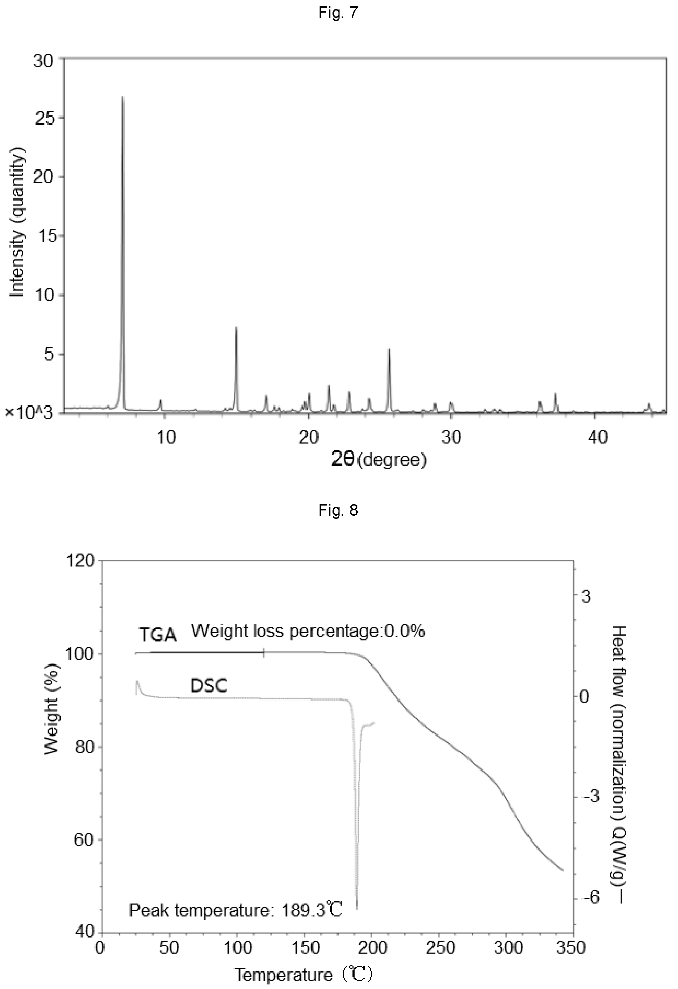

In another preferred embodiment, the crystal form Form B also has one or more features selected from the following groups.
(j1) An XRPD diagram of the crystal form Form B comprises 6 or more than 6 2θ values selected from the following groups: 5.3°±0.2°, 7.5°±0.2°, 8.1°±0.2°, 8.5°±0.2°, 9.6°±0.2°, 14.4°±0.2°, 16.0°±0.2°, 18.7°±0.2°, 19.2°±0.2°, 22.1°±0.2°, 22.8°±0.2°, 24.1°±0.2°, and 25.9°±0.2°.
(j2) The XRPD diagram of the crystal form Form B is characterized in Fig. 7.
(j3) The crystal form Form B loses 2.9% weight at 20-150 °C.
(j4) The TGA diagram of the crystal form Form B is characterized in Fig. 8.
(j5) Peak temperatures of a DSC diagram of the crystal form Form B are 57.2 °C, 146.6 °C, and 178.4 °C.
(j6) The DSC diagram of the crystal form Form B is characterized in Fig. 8.

In another preferred embodiment, n in the crystal form Form B is =0.5.

In another preferred embodiment, an XRPD diagram of the crystal form Form C includes 3 or more than 3 (4, 5, or 6) 2θ values selected from the following groups: 7.1°±0.2°, 9.7°±0.2°, 15.0°±0.2°, 21.5°±0.2°, 22.9°±0.2°, and 25.7°±0.2°.

In another preferred embodiment, the XRPD diagram of the crystal form Form C includes 1 or more than 1 2θ values selected from the following groups: 7.1°±0.2°, 9.7°±0.2°, 15.0°±0.2°, 21.5°±0.2°, 22.9°±0.2°, and 25.7°±0.2°.

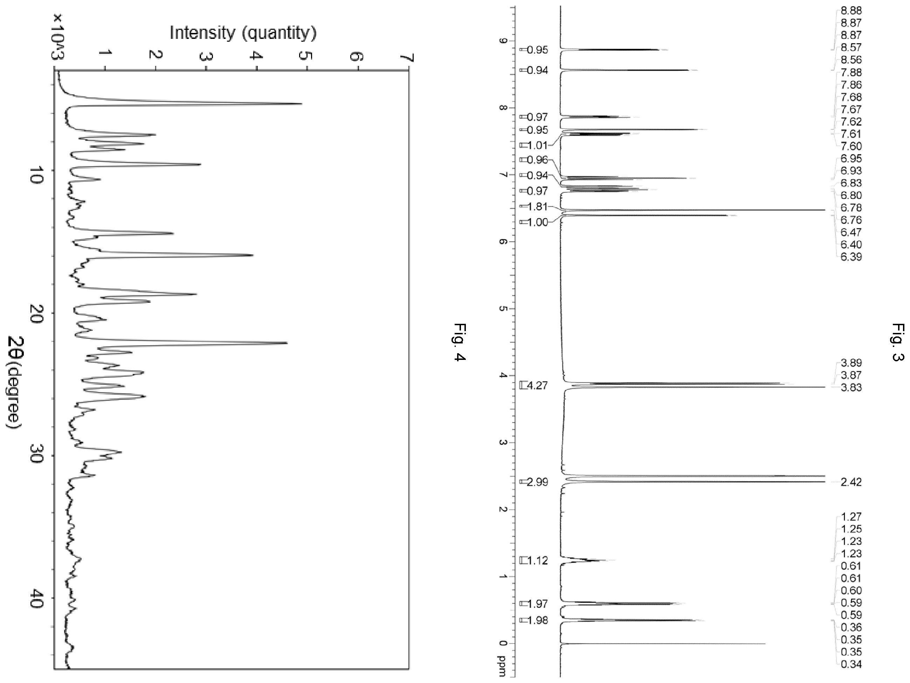

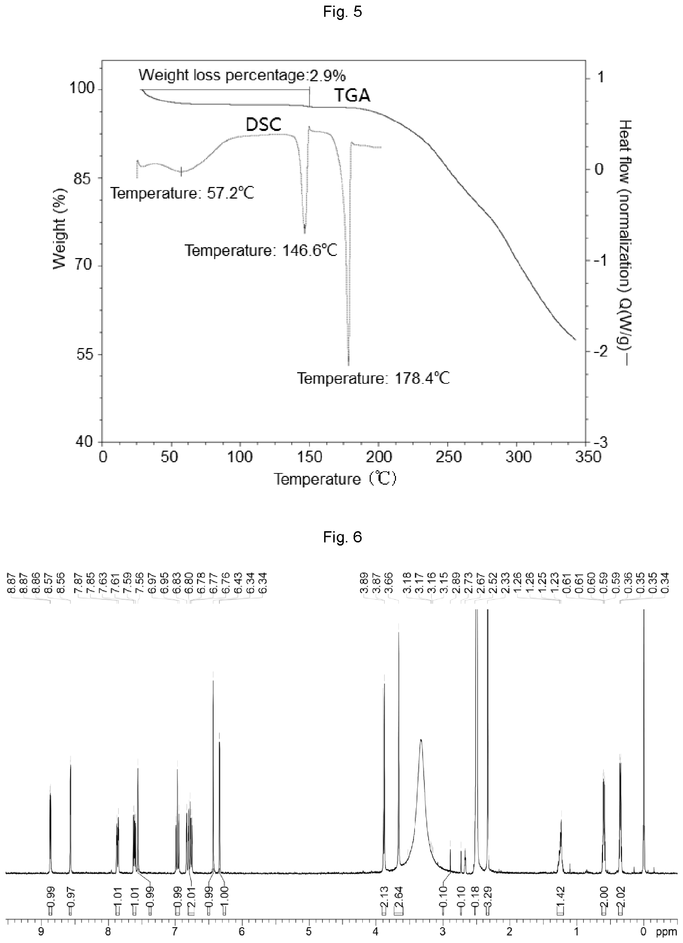

In another preferred embodiment, the crystal form Form C also has one or more features selected from the following groups.
(k1) The XRPD diagram of the crystal form Form C includes 6 or more than 6 2θ values selected from the following groups: 7.1°±0.2°, 9.7°±0.2°, 15.0°±0.2°, 17.1°±0.2°, 20.1°±0.2°, 21.5°±0.2°, 22.9°±0.2°, 23.8°±0.2°, 25.7°±0.2°, 30.0°±0.2°, and 37.3°±0.2°.
(k2) The XRPD diagram of the crystal form Form C is characterized in Fig. 4.
(k3) The crystal form Form C loses no weight at 20-120 °C.
(k4) A TGA diagram of the crystal form Form C is characterized in Fig. 5.
(k5) A peak temperature of a DSC diagram of the crystal form Form C is 189.3 °C.
(k6) The DSC diagram of the crystal form Form C is characterized in Fig. 5.

In another preferred embodiment, n in the crystal form Form C is =1.5.

In another preferred embodiment, an XRPD diagram of the crystal form Form D includes 3 or more than 3 (4, 5, or 6) 2θ values selected from the following groups: 7.6°±0.2°, 11.7°±0.2°, 19.8°±0.2°, 23.9°±0.2°, and 24.6°±0.2°.

In another preferred embodiment, the XRPD diagram of the crystal form Form D includes 1 or more than 1 2θ values selected from the following groups: 7.6°±0.2°, 11.7°±0.2°, and 19.8°±0.2°.

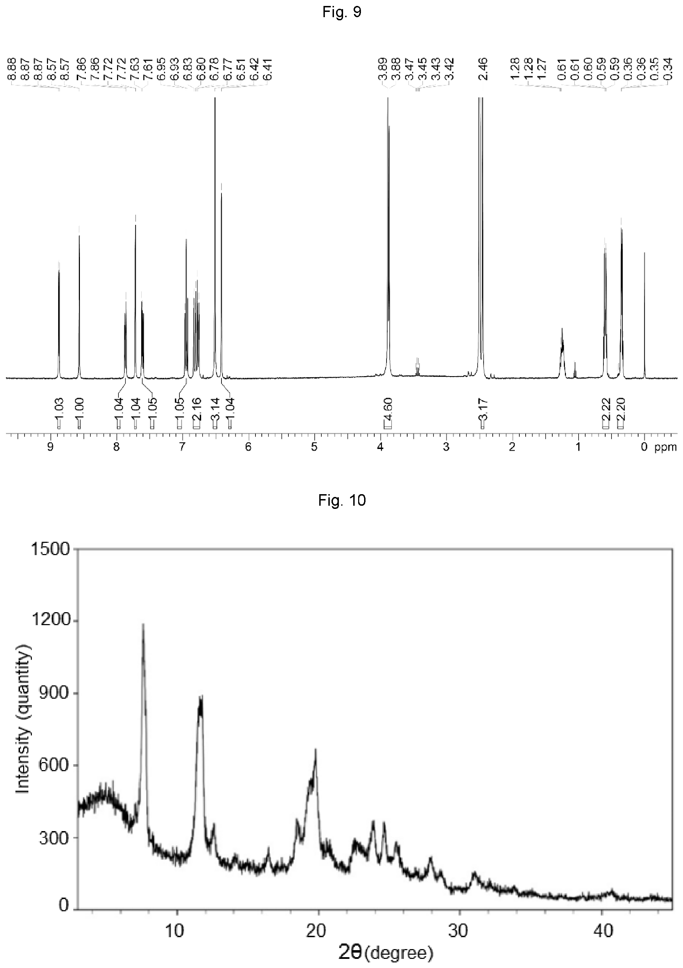

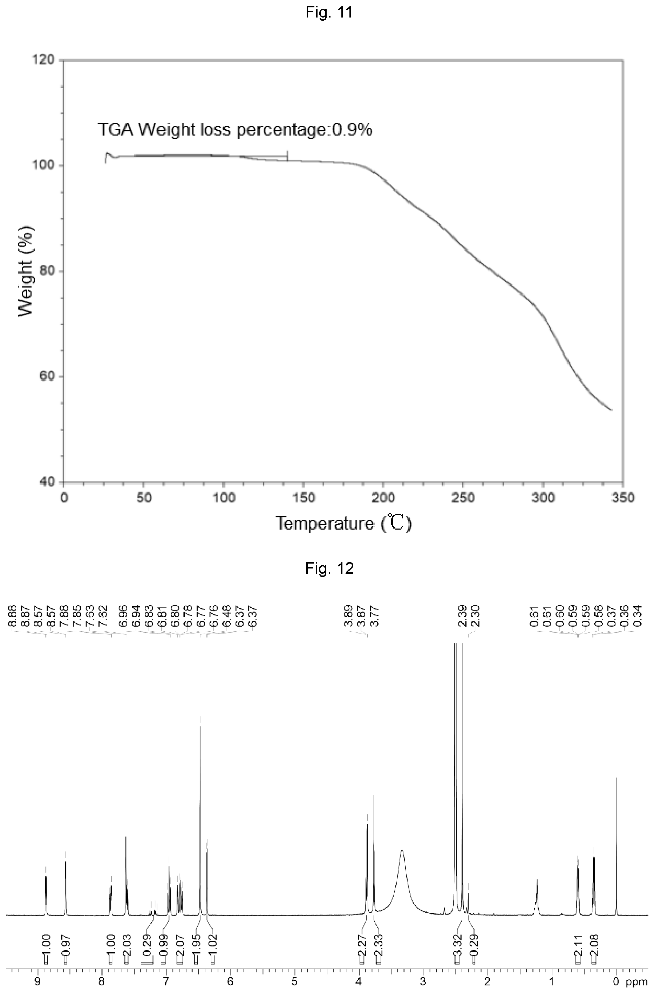

In another preferred embodiment, the crystal form Form D also has one or more features selected from the following groups.
(m1) The XRPD diagram of the crystal form Form D includes 6 or more than 6 2θ values selected from the following groups: 7.6°±0.2°, 12.6°±0.2°, 11.7°±0.2°, 16.5°±0.2°, 19.8°±0.2°, 23.9°±0.2°, 24.6°±0.2°, and 28.0°±0.2°.
(m2) The XRPD diagram of the crystal form Form D is characterized in Fig. 10.
(m3) The crystal form Form D loses 0.9% weight at 20-140 °C.
(m4) A TGA diagram of the crystal form Form D is characterized in Fig. 11.

In another preferred embodiment, n in the crystal form Form D is =1.

A second aspect of the present disclosure provides a method for preparing the crystal of the first aspect of the present disclosure. The crystal is of a crystal form Form A. The preparation method includes the following steps.

At (a1), a first substance is taken, where the first substance is 1-[5-[4-(cyclopropylmethoxy)-2-fluorophenyl]-1-(pyridin-3-ylsulfonyl)-1H-pyrrole-3-yl]-N-methylmeth anamine.

At (a2), the first substance is mixed with an organic solvent and fumaric acid for crystallization, so as to obtain the crystal form Form A.

A molar ratio of the first substance to the fumaric acid is 1:0.5-1.5.

In another preferred embodiment, the organic solvent is selected from the following groups: ethyl acetate, methyl acetate, butyl acetate, methanol, and ethanol or a combination thereof.

In another preferred embodiment, a mass ratio of the first substance to the fumaric acid is 10-3:1, preferably 5-3:1, and more preferably 4-3:1.

In another preferred embodiment, in step (a2), a mass-volume ratio of the first substance to the organic solvent is 1:5-30, preferably 1:10-25, and more preferably 1:10-20.

In another preferred embodiment, the method further includes a post-treatment step.

In another preferred embodiment, the post-treatment step includes filtration, washing, and drying.

In another preferred embodiment, the washing is performed by using an organic solvent.

In another preferred embodiment, vacuum drying is performed at 40-70 °C.

In another preferred embodiment, a drying time is 2-7 h, preferably 3-6 h.

A third aspect of the present disclosure provides a method for preparing the crystal of the first aspect of the present disclosure. The crystal is of a crystal form Form B. The method includes the following steps: a Form A is placed in a liquid-phase vial, a pre-heated methyl tert-butyl ether solution is added dropwise to form suspension, then pre-heated DMF is added dropwise, the mixture is transferred and cooled at -20 °C-30 °C, centrifugation is performed, and then a solid is dried to obtain the crystal form Form B.

A fourth aspect of the present disclosure provides a method for preparing the crystal of the first aspect of the present disclosure. The crystal is of a crystal form Form C. The method includes any one of steps (c1) and (c2).

At (c1), a first substance is mixed with an organic solvent and fumaric acid to obtain the crystal form Form C.

The first substance is 1-[5-[4-(cyclopropylmethoxy)-2-fluorophenyl]-1-(pyridin-3-ylsulfonyl)-1H-pyrrole-3-yl]-N-methylmeth anamine, and a molar ratio of the first substance to the fumaric acid is 1:2.5-4.0.

At (c2), a crystal form Form A is mixed with the organic solvent and the fumaric acid at 20-70 °C, so as to obtain the crystal form Form C.

In another preferred embodiment, the organic solvent is selected from the following groups: ethyl acetate, methyl acetate, butyl acetate, methanol, and ethanol or a combination thereof.

In another preferred embodiment, in step (c1), a mass ratio of the first substance to the fumaric acid is 3-0.5:1, preferably 2-1:1.

In another preferred embodiment, in step (c1), a mass-volume ratio of the first substance to the organic solvent is 1:5-30, preferably 1:10-25, and more preferably 1:10-20.

In another preferred embodiment, in step (c2), a molar ratio of the crystal form Form A to the fumaric acid is 1:1-1:5, preferably 1:1-1:2.

In another preferred embodiment, in step (c2), the organic solvent includes methanol and ethanol.

A fifth aspect of the present disclosure provides a method for preparing the crystal of the first aspect of the present disclosure. The crystal is of a crystal form Form D. The method includes the following steps: a crystal form Form C is placed in a glass vial, methanol is added for dissolving by ultrasonication, then toluene is added, the mixture is stirred and transferred to -20 °C-0 °C for standing for 1-5 days, and liquid is removed to obtain the crystal form Form D.

A six aspect of the present disclosure provides a drug composition. The drug composition contains: (a) any one of the above crystals shown in the Formula I, and (b) a pharmaceutically acceptable ingredients or carrier.

A seventh aspect of the present disclosure provides an application of any one of the crystals of the first aspect of the present disclosure. The crystal is used for preparing a Potassium Competitive Acid Blocker, and is used for preparing a drug or drug composition for treating erosive esophagitis, gastric ulcer, duodenal ulcer, indications for eradicating helicobacter pylori, and related diseases caused by excessive stomach acid.

It should be understood that, within the scope of the present disclosure, each of the above technical features of the present disclosure and each of the technical features specifically described below (for example, embodiments) can be combined with each other, so as to constitute new or preferred technical solutions. Due to the limitation of space, details are not described herein again.

### Brief Description of the Drawings

Fig. 1 shows an XRPD diagram of a crystal form Form A of a compound according to the present disclosure.
Fig. 2 shows TGA and DSC diagrams of a crystal form Form A of a compound according to the present disclosure.
Fig. 3 shows a Nuclear Magnetic Resonance (NMR) diagram of a crystal form Form A of a compound according to the present disclosure.
Fig. 4 shows an XRPD diagram of a crystal form Form B of a compound according to the present disclosure.
Fig. 5 shows TGA and DSC diagrams of a crystal form Form B of a compound according to the present disclosure.
Fig. 6 shows an NMR diagram of a crystal form Form B of a compound according to the present disclosure.
Fig. 7 shows an XRPD diagram of a crystal form Form C of a compound according to the present disclosure.
Fig. 8 shows TGA and DSC diagrams of a crystal form Form C of a compound according to the present disclosure.
Fig. 9 shows an NMR diagram of a crystal form Form C of a compound according to the present disclosure.
Fig. 10 shows an XRPD diagram of a crystal form Form D of a compound according to the present disclosure.
Fig. 11 shows a TGA diagram of a crystal form Form D of a compound according to the present disclosure.
Fig. 12 shows an NMR diagram of a crystal form Form D of a compound according to the present disclosure.
Fig. 13 shows a relationship diagram of solid-state conversion of a compound according to the present disclosure.
Fig. 14 shows a comparative XRPD diagram of different salt forms and crystal forms of a compound according to the present disclosure.

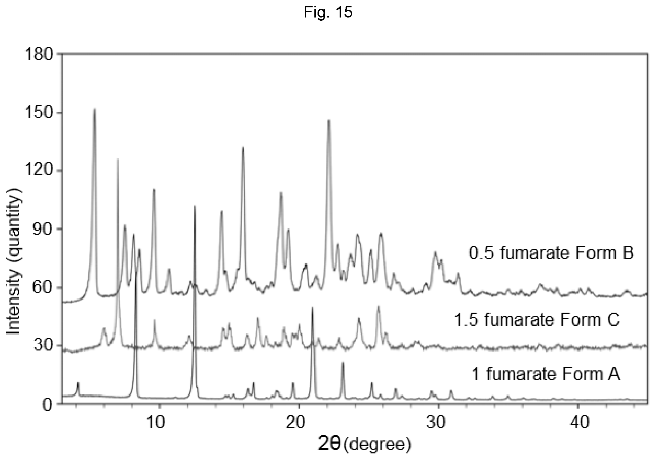

Fig. 15 shows a comparative XRPD diagram of different ratios of fumarate of a compound according to the present disclosure.
Fig. 16 shows (a) a DVS curve; and (b) an XRPD diagram before and after a DVS test of a crystal form Form A of a compound according to the present disclosure.
Fig. 17 shows a PLM image of a crystal form Form A of a compound according to the present disclosure.
Fig. 18 shows an XRPD diagram of a stability study of a crystal form Form A of a compound according to the present disclosure.
Fig. 19 shows an XRPD diagram of a stability study of a crystal form Form C of a compound according to the present disclosure.
Fig. 20 shows a comparative XRPD diagram of the remaining solid of a crystal form Form A of a compound after 24h of oscillation in a medium according to the present disclosure.

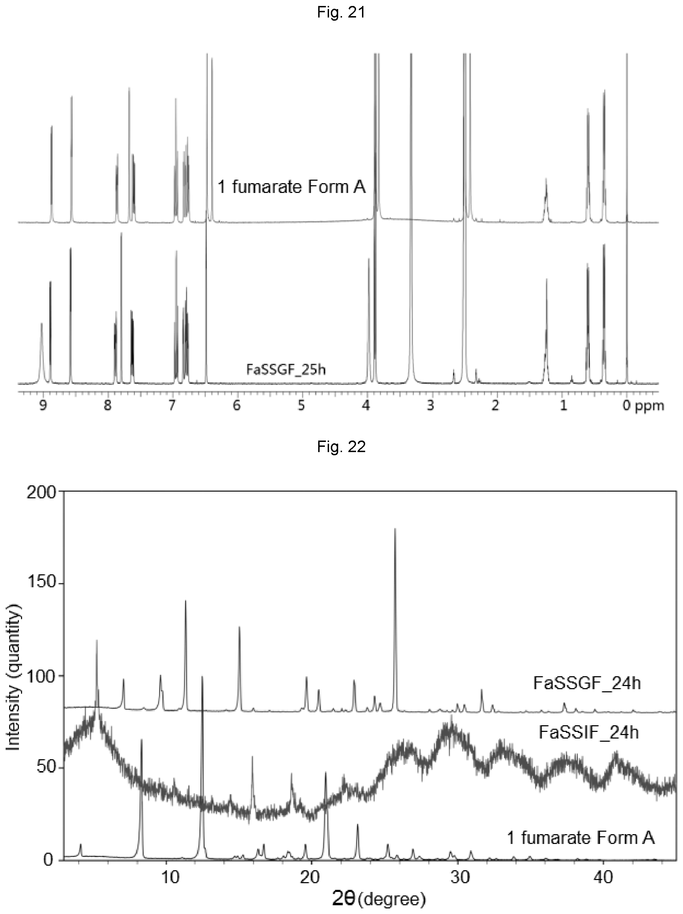

Fig. 21 shows a comparative NMR diagram of the remaining solid of a crystal form Form A of a compound after 24h of oscillation in FaSSGF according to the present disclosure.
Fig. 22 shows a comparative XRPD diagram of reproducing the remaining solid of a crystal form Form A of a compound after 24h of oscillation in FaSSIF and FaSSGF according to the present disclosure.

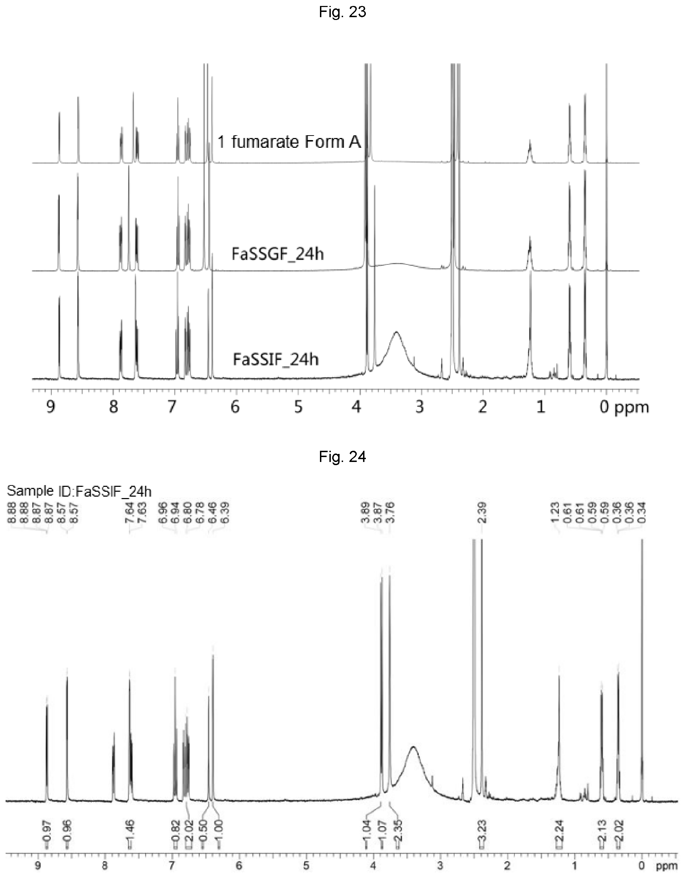

Fig. 23 shows a comparative NMR diagram of reproducing the remaining solid of a crystal form Form A of a compound after 24h of oscillation in FaSSIF and FaSSGF according to the present disclosure.
Fig. 24 shows a comparative NMR diagram of reproducing the remaining solid of a crystal form Form A of a compound after 24h of oscillation in FaSSIF according to the present disclosure.

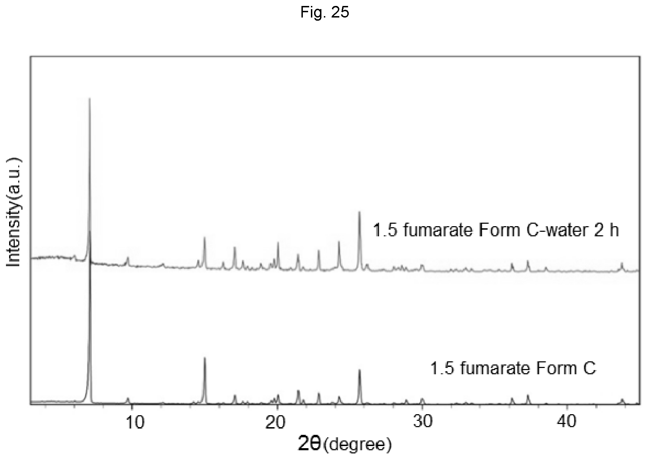

Fig. 25 shows a comparative XRPD diagram (normalization processing) of the remaining solid of a crystal form Form C of a compound after 2h of oscillation in water according to the present disclosure.
Fig. 26 shows (a) a DVS curve; and (b) an XRPD diagram before and after a DVS tests of a crystal form Form C of a compound according to the present disclosure.
Fig. 27 shows an overlay comparison diagram of thermal crystal transformation of a crystal form Form B of a compound according to the present disclosure.
Fig. 28 shows a comparative XRPD diagram of the remaining solid of crystal forms Form B and Form A of a compound after 2h of oscillation in water according to the present disclosure.
Fig. 29 shows (a) a DVS curve; and (b) an XRPD diagram before and after a DVS tests of a crystal form Form B of a compound according to the present disclosure.

### Detailed Description of the Embodiments

The present inventors have provided a salt crystal form of a compound of a Formula I after a long and intensive study. These crystal forms have advantages in terms of stability, solubility, hygroscopicity, mechanical stability, tablet stability, fluidity, process exploitability, preparation research and development and powder processing performance, etc. In particular, crystal forms Form A and Form C have significant advantages in preparation process and stability. On the basis of the above findings, the inventor has completed the present disclosure.

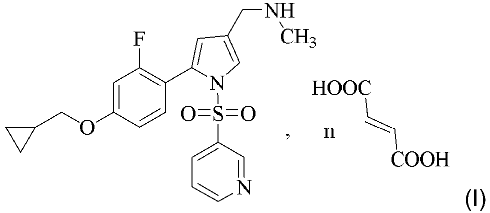

### Terms

**In** the present specification, unless otherwise noted, each abbreviation has a conventional meaning as understood by those skilled in the art.

As used herein, unless otherwise indicated, the solvent or solution is directly added or added at a constant speed, etc.

As used herein, the term "room temperature" generally refers to 4 to 30 °C, preferably 20±5 °C.

As used herein, "slow addition" includes, but is not limited to, dropwise addition, slow addition along the wall of a container, and the like.

As used herein, the terms "containing" or "comprising (including)" may be open, semi-closed, and closed. **In** other words, the term also includes "composed essentially of", or "composed of".

As used herein, the term "m or more than m 2θ values selected from the following group" refers to any positive integer (e.g., m, m+1, ...) including n and greater than n, where an upper limit, Nup, is the number of all 2θ peak values in the group. For example, "3 or more than 3" not only includes 3, 4, 5, 6, 7, 8, 9, 10, 11, 12, 13, 14, 15, 16, 17, 18, 19, 20, 21, ..., the upper limit Nup for all positive integers, but also includes ranges such as "4 or more than 4", "5 or more than 5", "6 or more than 6", etc.

As used herein, the terms "compound of a Formula I", "raw material", "fumarate of the compound of the Formula I", "salt of a pyrrole sulfonamide acid inhibitor", "1-[5-[4-(cyclopropylmethoxy)-2-fluorophenyl]-1-(pyridin-3-ylsulfonyl)-1H-pyrrole-3-yl]-N-methylmet hanamine fumarate", and "compound of the present disclosure" may be used interchangeably and refer to the compounds shown in the Formula I, where n=0.1-2.0.

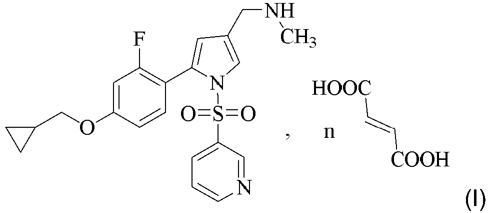

As used herein, the "N-methylmethanamine fumarate" and "n" may be used interchangeably and refer to the number of methylmethanamine fumarates bound in the compound shown in the Formula I. For example, n=1, that is, 1-[5-[4-(cyclopropylmethoxy)-2-fluorophenyl]-1-(pyridin-3-ylsulfonyl)-1H-pyrrole-3-yl]-1-methylmeth anamine fumarate.

As used herein, "crystal form Form A", "Form A", "raw material (Form A)", and "1 fumarate Form A" may be used interchangeably and all refer to the crystal form Form A of the compound shown in the Formula I. As used herein, "crystal form Form B", "Form B", and "0.5 fumarate Form B" may be used interchangeably and all refer to the crystal form Form B of the compound shown in the Formula I. As used herein, "crystal form Form C", "Form C", and "1.5 fumarate Form C" may be used interchangeably and all refer to the crystal form Form C of the compound shown in the Formula I. As used herein, "crystal form Form D" and "Form D" may be used interchangeably and all refer to the crystal form Form D of the compound shown in the Formula I.

### Drug composition containing crystal form of compound of Formula I

Another aspect of the present disclosure provides a drug composition, which contains a therapeutically effective amount of a crystal form of a compound of Formula I of the present disclosure, and optionally, one or more pharmaceutically usable carriers, Binders, adjuvants, excipients and/or diluents. The excipients are, for example, odorants, flavoring agents, sweetening agents, etc.

The drug composition provided in the present disclosure preferably contains an active ingredient in a weight ratio of 1-99%, with a preferred ratio of 65 wt%-99 wt% of a total weight of the compound of general formula I as the active ingredient, and the rest is a pharmaceutically acceptable carrier, diluent or solution or salt solution. The compound and drug composition provided in the present disclosure may be in a variety of forms, such as tablets, capsules, powders, syrups, solutions, suspensions, aerosols, etc., and may be present in suitable solid or liquid carriers or diluents and in suitable sterilized instruments for injection or infusion.

Various dosage forms of the drug composition of the present disclosure may be prepared according to conventional preparation methods in the field of pharmacology. A formulation of the preparation contains 1 mg-700 mg of the compound of the general formula I in unit measurement, preferably, the formulation of the preparation contains 25 mg-300 mg of the compound of the general formula I in unit measurement.

The compound and drug composition of the present disclosure are available for clinical use in mammals, including humans and animals, and may be administered through routes such as the mouth, nose, skin, lung, gastrointestinal tract, or the like, most preferably orally. The most preferred daily dose is 50-1400 mg/kg body weight in a single dose, or 25-700 mg/kg body weight in divided doses. Regardless of the method of administration, the optimal dose for an individual should be based on specific treatments. It is usual to start with a small dose and gradually increase the dose until the most suitable dose is found.

In the present disclosure, unless otherwise indicated, the method used for drying is a conventional drying method in the art, for example, in embodiments of the present disclosure, drying means vacuum drying or atmospheric pressure drying in an oven for conventional drying. Generally, drying is performed for 0.1-50 h or 1-30 h.

### The present disclosure has the following main advantages.

(1) Compared with an amorphous solid thereof, the compound crystal (including the crystal form Form A, crystal form Form B, crystal form Form C, and crystal form Form D) of the present disclosure is high in purity, good in stability, good in fluidity, and low in moisture absorption.
(2) The method for preparing the compound crystal (including the crystal form Form A, crystal form Form B, crystal form Form C, and crystal form Form D) of the present disclosure is simple, and compared with a freeze-drying process (large in energy consumption and limited in capacity), the preparation method is more suitable for industrial production.
(3) The compound crystal (including the crystal form Form A, crystal form Form B, crystal form Form C, and crystal form Form D) of the present disclosure may be used to prepare a Potassium Competitive Acid Blocker, and is used for preparing drugs for treating erosive esophagitis, gastric ulcer, duodenal ulcer, indications for eradicating helicobacter pylori, and related diseases caused by excessive stomach acid.

The present disclosure is further described below with reference to the specific embodiments. It should be understood that, these embodiments are only intended to describe the present disclosure and are not intended to limit the scope of the present disclosure. Experimental methods for which specific conditions are not indicated in the following embodiments usually follow conventional conditions, or follow the conditions recommended by manufacturers. Percentages and parts are calculated by weight unless otherwise specified.

Unless otherwise defined, all professional and scientific terms used in the text have the same meaning as those familiar to those skilled in the art. Furthermore, any methods and materials that are similar or equivalent to what is documented may be applied to the inventive methods. The preferred implementations and materials in the text are for demonstration purposes only.

### Generic method and reagent

Solvents used in the present disclosure are all analytically pure.

All test methods of the present disclosure are all generic methods, and test parameters are as follows.

### Nuclear magnetic analysis (¹H NMR)

Several milligrams of a solid sample are dissolved in a dimethyl sulfoxide-d6 solvent, and nuclear magnetic analysis is performed on Bruker AVANCE NEO 400 (Bruker, GER).

### X-ray Powder Diffraction (XRPD)

An X-ray Powder Diffraction instrument Malvern PANalytical Aeris (Malvern Panalytical, UK) is used for a preliminary test, a 2θ scanning angle is from 3° to 45°, a scanning step size is 0.02°, and a test time is 4.9 min. A light tube voltage and current are respectively 40 kV and 7.5 mA when the sample is tested, and a sample disk is a zero background sample disk. Alternatively, an X-ray Powder Diffraction instrument Panalytical EMPYREAN(PANalytical, UK) is used again for characterization. A 2θ scanning angle is from 3° to 45°, a scanning step size is 0.013°, and a test time is 3.5 min. A test method includes a Cu target K α 1 ray, a voltage being 45 kV, a current being 40 mA, and a sample disk is a zero background sample disk.

### Online variable temperature XRPD test

The X-ray Powder Diffraction instrument Malvern PANalytical Aeris (Malvern Panalytical, UK) is used, the 2θ scanning angle is from 3° to 40°, the scanning step size is 0.02°, and the test time is 13 min. The light tube voltage and current are respectively 40 kV and 7.5 mA when the sample is tested, and the sample disk is the zero background sample disk. The sample is placed on a BTS500 heating stage (Anton Paar, AT), an XRPD test is performed at room temperature, then a temperature is heated at 10 °C/min to a selected temperature, the XRPD test is performed at temperature after 10 min of isothermalization, and then the XRPD test is performed by cooling the temperature to room temperature.

### Thermogravimetric Analysis (TGA)

A model of a Thermogravimetric Analyzer is TA Discovery 55(TA, US). 2-5 mg of the sample is placed in an equilibrated open aluminum sample disk, and is automatically weighed in a TGA heating furnace. The sample is heated to a final temperature at a rate of 10 °C/min, a speed for nitrogen purging at the sample is 60 mL/min, and a speed for nitrogen purging at a balance is 40 mL/min.

### Differential Scanning Calorimetry (DSC) analysis

A model of a **Differential Scanning Calorimetry** analyzer is TA Discovery 2500(TA, US). 1-2 mg of the sample is accurately weighed and then placed in a DSC Tzero sample with pierced holes, and heated to the final temperature at the rate of 10 °C/min, and a speed for nitrogen purging in the furnace is 50 mL/min.

### Dynamic moisture adsorption and desorption analysis (DVS)

Dynamic moisture adsorption and desorption analysis for preliminary assessment of hygroscopicity is determined by using DVS Intrinsic Plus (SMS, UK). The test uses a gradient mode, a humidity change is 50%-95%-50%, a humidity change amount of each gradient is 15%, a gradient end is determined by using a dm/dt mode, and dm/dt being less than 0.002% and maintained for 10 minutes is used as the gradient end, or the longest maintenance time of each gradient is 60 min. After the test is completed, XRPD analysis is performed on the sample to confirm whether a solid form changes.

The dynamic moisture adsorption and desorption analysis is determined by using the DVS Intrinsic Plus (SMS, UK). The test uses the gradient mode, the humidity change is 0%-95%-0%, the humidity change amount of each gradient is 10% within a range of 0% to 90%, the gradient end is determined by using the dm/dt mode, and dm/dt being less than 0.002% and maintained for 10 minutes is used as the gradient end, or the longest maintenance time of each gradient is 180 min. After the test is completed, XRPD analysis is performed on the sample to confirm whether the solid form changes.

### High-Performance Liquid Chromatography (HPLC)

An **High-Performance Liquid Chromatography** model is SHIMADZU LC-20A (Shimadzu, JP), and test conditions are shown in Table 0 below.

**Table 0 HPLC test conditions**

| | | | | |
|---|---|---|---|---|
| Chromatographic column | Shim-pack GIST, 4.6×250 mm(HSS), 5 µm, C18 | | | |
| Mobile phase | A: 0.01M potassium hexafluorophosphate (1 mL of trifluoroacetic acid is added in per L of a solution) | | | |
| | B: Acetonitrile | | | |
| | D: Methanol | | | |
| Gradient | Time (min) | Mobile phase A (%) | Mobile phase B (%) | Mobile phase D (%) |
| | 0 | 94 | 3 | 3 |
| | 1 | 94 | 3 | 3 |
| | 3 | 69 | 3 | 28 |
| | 10 | 44 | 28 | 28 |
| | 30 | 44 | 28 | 28 |
| | 30.1 | 94 | 3 | 3 |
| | 45 | 94 | 3 | 3 |
| Flow rate | | 0.8 mL/min | | |
| Detection wavelength | | 225 nm | | |
| Column temperature | | 35 °C | | |
| Diluent | | 90% methanol aqueous solution | | |

### Embodiment 1 Preparation of crystal form Form A

### 1.1. Synthesis method

1-[5-[4-(cyclopropylmethoxy)-2-fluorophenyl]-1-(pyridin-3-ylsulfonyl)-1H-pyrrole-3-yl]-N-methyl methanamine (2.00 g), ethyl acetate (30 mL), and fumaric acid (0.67 g, 1.2 eq.) were added in a 100 mL single-neck, stirred at room temperature for 0.5 h, filtered, washed with ethyl acetate (6 mL), and dried in vacuum for 3 h at 50 °C, so as to obtain 2.25 g of a white solid, with a yield of 88%.

The 1-[5-[4-(cyclopropylmethoxy)-2-fluorophenyl]-1-(pyridin-3-ylsulfonyl)-1H-pyrrole-3-yl]-N-methylmeth anamine (2.00 g), ethanol (20 mL), and the fumaric acid (0.50 g, 0.9 eq.) were added in the 100 mL single-neck, stirred at room temperature for 1 h, filtered, washed with ethanol (10 mL), and dried in vacuum for 5 h at 45 °C, so as to obtain 1.80 g of a white solid, with a yield of 78%.

The 1-[5-[4-(cyclopropylmethoxy)-2-fluorophenyl]-1-(pyridin-3-ylsulfonyl)-1H-pyrrole-3-yl]-N-methylmeth anamine (2.00 g), the ethyl acetate (40 mL), and the fumaric acid (0.56 g, 1.0 eq.) were added in the 100 mL single-neck, stirred at room temperature for 1 h, filtered, washed with ethyl acetate (10 mL), and dried in vacuum for 6 h at 60 °C, so as to obtain 2.15 g of the white solid, with the yield of 84%.

### 1.2 Thermal crystal transformation method

Thermal crystal transformation was performed on an online variable temperature X-ray Powder Diffraction instrument Malvern PANalytical Aeris(Malvern Panalytical, UK). The sample was placed on a BTS500 heating stage (Anton Paar, AT), an XRPD test was performed at room temperature, then a temperature was heated at 10 °C/min to a selected temperature, the XRPD test was performed at temperature after 10 min of isothermalization, and then the XRPD test was performed by cooling the temperature to room temperature. Diagram data was shown in Table 1. (Hereinafter referred to as an online variable temperature XRPD experiment)

**Table 1 XRPD table of crystal form Form A**

| 2θ | d Value [Å] | Relative intensity [%] |
|---|---|---|
| 4.15 | 21.27993 | 9.3 |
| 8.33 | 10.63772 | 65.8 |
| 11.14 | 7.97596 | 1.9 |
| 12.50 | 7.11650 | 100.0 |
| 14.72 | 6.06244 | 2.7 |
| 14.92 | 5.98419 | 2.7 |
| 15.30 | 5.83868 | 3.4 |
| 16.32 | 5.48140 | 6.4 |
| 16.72 | 5.35580 | 9.0 |
| 17.65 | 5.08121 | 1.6 |
| 18.07 | 4.96687 | 2.7 |
| 18.36 | 4.89135 | 5.3 |
| 18.49 | 4.85782 | 4.9 |
| 18.69 | 4.80841 | 2.6 |
| 19.07 | 4.71586 | 1.7 |
| 19.57 | 4.60014 | 9.2 |
| 20.95 | 4.30965 | 46.2 |
| 23.14 | 3.92062 | 19.9 |
| 25.21 | 3.61661 | 9.3 |
| 25.83 | 3.53593 | 3.1 |
| 26.92 | 3.40284 | 6.5 |
| 27.38 | 3.35003 | 2.5 |
| 29.48 | 3.13054 | 5.1 |
| 29.78 | 3.10166 | 3.0 |
| 30.88 | 3.00127 | 5.4 |
| 32.17 | 2.89337 | 1.7 |
| 32.63 | 2.85703 | 1.4 |
| 33.83 | 2.76750 | 2.3 |
| 34.96 | 2.68895 | 2.4 |
| 36.03 | 2.61901 | 1.5 |

### Embodiment 2 Preparation of crystal form Form B

A preparation process was shown in Table 2. Form B was successfully prepared in scale-up preparation.

**Table 2 Scale-up preparation of target salt form**

| Process | Result |
|---|---|
| 200.3 mg of the crystal form Form A was weighed and added in 10.0 mL of MTBE at 50 °C, so as to form a suspension. 6.0 mL of pre-heated DMF was added dropwise until a solid was just about completely dissolved, and the solution was transferred to room temperature for cooling. The solution was allowed to stand for more than 2 h at room temperature, and then placed at -15 °C for cooling. After centrifugation, the solid is dried in vacuum at 40 °C. | 59.1 mg white powder Form B |
| 200.2 mg of the crystal form Form A was weighed and added in 10.0 mL of MTBE at 50 °C, so as to form a suspension. 5.0 mL of pre-heated DMF was added dropwise until a solid was just about completely dissolved, and the solution was filtered and then transferred to room temperature for cooling. The solution was allowed to stand for more than 2 h at room temperature, and then placed at -15 °C for cooling. After centrifugation, the solid is dried in vacuum at 40 °C. | 98.7 mg white powder Form B |

Table 3 was an XRPD table of crystal form Form B.

**Table 3 XRPD table of crystal form Form B**

| 2θ | d Value [Å] | Relative intensity [%] |
|---|---|---|
| 5.35 | 16.52912 | 100.0 |
| 7.52 | 11.76973 | 41.1 |
| 8.15 | 10.86519 | 36.3 |
| 8.54 | 10.37109 | 28.7 |
| 9.63 | 9.21000 | 58.7 |
| 10.65 | 8.33551 | 18.8 |
| 11.11 | 7.99705 | 6.7 |
| 11.52 | 7.71407 | 7.1 |
| 12.22 | 7.28102 | 12.0 |
| 12.45 | 7.14341 | 11.0 |
| 12.67 | 7.02285 | 10.1 |
| 13.28 | 6.70645 | 7.9 |
| 14.45 | 6.17216 | 48.2 |
| 14.76 | 6.04766 | 17.1 |
| 15.98 | 5.59760 | 80.3 |
| 16.39 | 5.46029 | 13.8 |
| 16.82 | 5.32313 | 12.3 |
| 17.15 | 5.22490 | 9.1 |
| 17.65 | 5.08143 | 10.0 |
| 17.97 | 4.99223 | 11.5 |
| 18.69 | 4.80714 | 57.5 |
| 19.24 | 4.67629 | 38.7 |
| 20.50 | 4.39996 | 21.0 |
| 21.17 | 4.26606 | 14.7 |
| 22.15 | 4.08655 | 94.2 |
| 22.80 | 3.97566 | 31.6 |
| 23.17 | 3.91567 | 17.7 |
| 23.69 | 3.83427 | 26.4 |
| 24.15 | 3.76602 | 35.8 |
| 25.10 | 3.63134 | 26.5 |
| 25.86 | 3.53158 | 37.1 |
| 26.78 | 3.41965 | 16.2 |
| 27.15 | 3.37657 | 11.8 |
| 28.15 | 3.26594 | 8.9 |
| 29.12 | 3.16541 | 11.6 |
| 29.75 | 3.10431 | 27.3 |
| 30.23 | 3.05975 | 23.5 |
| 31.38 | 2.95830 | 16.6 |
| 32.28 | 2.88510 | 8.2 |
| 33.17 | 2.81610 | 7.1 |
| 35.01 | 2.68505 | 8.0 |
| 35.90 | 2.62706 | 7.8 |
| 37.25 | 2.54510 | 10.9 |
| 37.99 | 2.50286 | 8.5 |
| 38.49 | 2.47531 | 8.7 |
| 40.10 | 2.39183 | 8.7 |
| 40.73 | 2.36113 | 9.1 |
| 43.55 | 2.23585 | 7.1 |

### Embodiment 3 Preparation of crystal form Form C

### 3.1. Synthesis method

1-[5-[4-(cyclopropylmethoxy)-2-fluorophenyl]-1-(pyridin-3-ylsulfonyl)-1H-pyrrole-3-yl]-N-methyl methanamine (2.00 g), ethyl acetate (40 mL), and fumaric acid (1.40 g, 2.5 eq.) were added in a 100 mL single-neck, stirred at room temperature for 0.5 h, filtered, washed with ethyl acetate (10 mL), and dried in vacuum for 6 h at 45 °C, so as to obtain 2.41 g of a white solid, with a yield of 85%.

The 1-[5-[4-(cyclopropylmethoxy)-2-fluorophenyl]-1-(pyridin-3-ylsulfonyl)-1H-pyrrole-3-yl]-N-methylmeth anamine (2.00 g), methanol (30 mL), and the fumaric acid (1.68 g, 3.0 eq.) were added in the 100 mL single-neck, stirred at room temperature for 1 h, filtered, washed with methanol (10 mL), and dried in vacuum for 6 h at 50 °C, so as to obtain 2.47 g of a white solid, with a yield of 87%.

The 1-[5-[4-(cyclopropylmethoxy)-2-fluorophenyl]-1-(pyridin-3-ylsulfonyl)-1H-pyrrole-3-yl]-N-methylmeth anamine (2.00 g), ethanol (30 mL), and the fumaric acid (1.96 g, 3.5 eq.) were added in the 100 mL single-neck, stirred at room temperature for 0.5 h, filtered, washed with ethanol (10 mL), and dried in vacuum for 6 h at 60 °C, so as to obtain 2.44 g of a white solid, with a yield of 86%.

The ethanol (20 mL) and the fumaric acid (0.66 g, 1.5 eq.) were added in the 100 mL single-neck and dissolved through heating, the 1-[5-[4-(cyclopropylmethoxy)-2-fluorophenyl]-1-(pyridin-3-ylsulfonyl)-1H-pyrrole-3-yl]-N-methylmeth anamine monofumarate (2.00 g) was added, and the mixture was stirred for 0.5 h, filtered, washed with ethanol (10 mL), and dried in vacuum for 4 h at 50 °C, so as to obtain 2.04 g of the white solid, with a yield of 92%.

XRPD data of the crystal form Form C was shown in Table 4.

**Table 4 XRPD table of crystal form Form C**

| 2θ | d Value [Å] | Relative intensity [%] |
|---|---|---|
| 6.03 | 14.66719 | 2.3 |
| 7.08 | 12.49976 | 100.0 |
| 9.73 | 9.11378 | 4.5 |
| 12.15 | 7.32092 | 1.6 |
| 14.24 | 6.26484 | 1.8 |
| 14.56 | 6.12659 | 1.8 |
| 15.01 | 5.94838 | 27.5 |
| 15.98 | 5.59533 | 1.1 |
| 16.28 | 5.49430 | 1.4 |
| 17.10 | 5.23997 | 6.1 |
| 17.66 | 5.07756 | 2.4 |
| 17.95 | 4.99848 | 2.1 |
| 18.33 | 4.89811 | 1.0 |
| 18.92 | 4.75052 | 1.5 |
| 19.59 | 4.59421 | 2.6 |
| 19.79 | 4.55030 | 3.9 |
| 20.07 | 4.49029 | 6.5 |
| 21.46 | 4.21148 | 8.9 |
| 21.80 | 4.14867 | 2.9 |
| 22.88 | 3.96317 | 7.1 |
| 23.79 | 3.81848 | 1.6 |
| 24.28 | 3.74657 | 5.3 |
| 25.69 | 3.55445 | 20.4 |
| 26.26 | 3.48163 | 1.4 |
| 28.05 | 3.27631 | 1.5 |
| 28.63 | 3.21562 | 1.4 |
| 28.89 | 3.18888 | 3.3 |
| 29.98 | 3.08316 | 3.7 |
| 30.06 | 3.07583 | 3.1 |
| 32.36 | 2.87870 | 1.6 |
| 33.04 | 2.82575 | 1.8 |
| 33.41 | 2.79820 | 1.3 |
| 36.20 | 2.60834 | 4.2 |
| 36.29 | 2.60264 | 2.5 |
| 37.29 | 2.54273 | 6.4 |
| 37.38 | 2.53739 | 3.3 |
| 43.54 | 2.23634 | 1.6 |
| 43.79 | 2.22616 | 3.4 |
| 43.91 | 2.22139 | 1.8 |
| 44.82 | 2.18576 | 1.3 |

### Embodiment 4 Preparation of crystal form Form D

19.6 mg of the crystal form Form C was weighed by using a preparation workstation ICSW-V3(XtalPi, CN) and was completely dissolved by dropwise adding 1.7 mL of methanol at room temperature, 15.0 mL of a toluene solution was then added and stirred for 1 h at room temperature, and the mixture was filtered and then transferred to -15 °C for standing for 1 day. The obtained solid was centrifuged and dried in vacuum at room temperature, and then an XRPD test is performed, as shown in Table 5.

Table 5 was an XRPD table of crystal form Form D.

**Table 5 XRPD table of crystal form Form D**

| 2θ | d Value [Å] | Relative intensity [%] |
|---|---|---|
| 7.62 | 11.62112 | 100.0 |
| 11.66 | 7.62140 | 73.6 |
| 12.63 | 7.04372 | 30.3 |
| 14.09 | 6.32773 | 19.9 |
| 16.47 | 5.43464 | 22.0 |
| 18.46 | 4.86448 | 31.6 |
| 19.79 | 4.55030 | 56.4 |
| 22.57 | 4.01336 | 24.9 |
| 23.90 | 3.80267 | 31.3 |
| 24.65 | 3.69410 | 30.6 |
| 25.53 | 3.57490 | 24.3 |
| 27.98 | 3.28338 | 18.9 |
| 28.60 | 3.21832 | 14.2 |
| 31.06 | 2.98641 | 13.6 |

### Embodiment 5 Characterization of crystal form

### 5.1 Crystal form Form A

The XRPD results showed that the Form A was a solid with good crystallinity. The TGA results showed that the Form A basically lost no weight when being heated to 150 °C, and might decompose above 195 °C. The DSC results showed that the Form A had a molten heat-absorption signal at about 188 °C. The NMR results showed that the sample was consistent with a reference diagram, with no obvious organic solvent signal peaks. The PLM image showed that the Form A was a bulk crystal, with a grain size being generally less than 25 µm. To sum up, the Form A was an anhydrous crystal form.

**Table 6 System characterization results for fumarate Form A**

| Crystal form | Form A | |
|---|---|---|
| Solvation information | anhydrate | |
| TGA Weight loss percentage%/temperature range °C | 0.0/25-150 | |
| DSC peak temperature °C | Heat absorption: 188.4 | |
| Hygroscopicity | Almost no hygroscopicity | |
| Purity % | 99.34 | |
| 60 °C | No change in appearance and XRPD/99.76 | |
| Physical stability/purity % for 15 days | | |
| 25 °C/92.5% RH | No change in appearance and XRPD/99.73 | |
| Physical stability/purity % for 15 days | | |
| 25 °C/4500 Lux | No change in appearance and XRPD/99.73 | |
| Physical stability/purity % for 15 days | | |
| 40 °C/75% RH | White powder turning yellow, no change in XRPD/98.76 | |
| Physical stability/purity % for 15 days | | |
| 24 h solubility (mg/mL) | FaSSIF | 1.99 |
| | FeSSIF | 2.88 |
| | FaSSGF | 3.78 |
| | Water | 3.17 |
| | 0.9% normal saline | 3.17 |
| | 5% glucose | 3.13 |
| Changes in XRPD of residual solids in 24 h solubility experiment | FaSSIF | Dissociation |
| | FeSSIF | Oily substance |
| | FaSSGF | Dissociation |
| | Water | Form A |
| | 0.9% normal saline | Form A |
| | 5% glucose | Form A |

### 5.2 Crystal form Form B

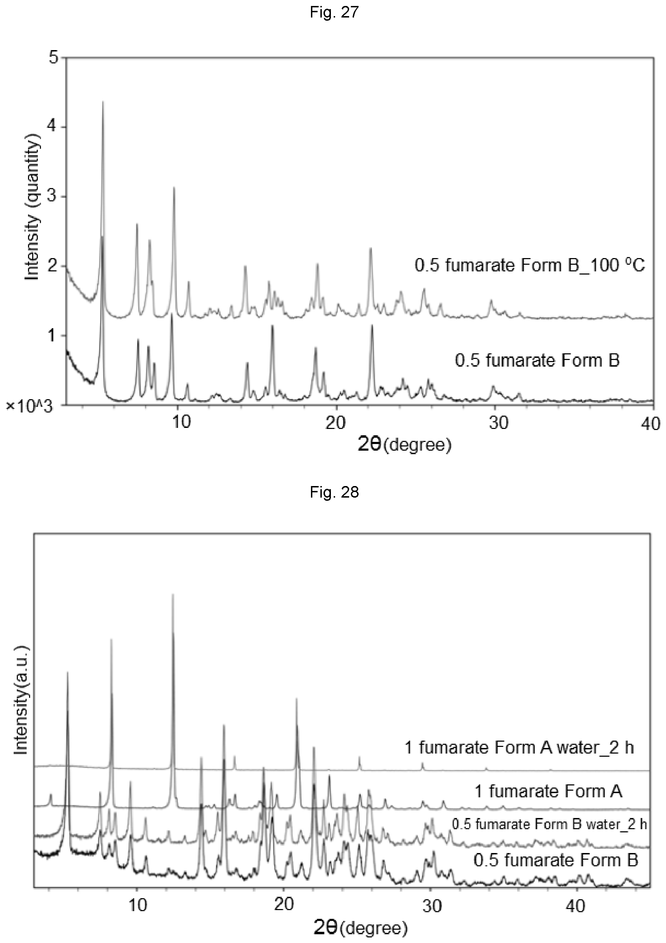

The XRPD results showed that the Form B was a solid with good crystallinity. The TGA results showed that the Form B lost 2.9% weight when being heated to 150 °C, and might decompose above 210 °C.The DSC results showed that the Form C had a wider heat-absorption signal corresponding to TGA weight loss at 40 °C to 90 °C, and had the heat-absorption signal at about 147 °C and about 178 °C. The thermal crystal transformation experiment results indicated that the XRPD did not change after the Form B was heated to 100 °C, as shown in Fig. 27. The NMR results showed that a structure of the compound did not change, a signal peak of the fumaric acid might be seen at 6.43 ppm, signal peaks of the DMF might be seen at 2.73 ppm and 2.89 ppm, there was a small amount of residual solvent, and according to an integral result, a ratio of the compound to the fumaric acid was calculated to be 1:0.5. To sum up, the Form B was an anhydrate.

### 5.3 Crystal form Form C

The XRPD results showed that the Form C was a solid with good crystallinity. The TGA results showed that the Form C lost no weight when being heated to 120 °C, and might decompose above 190 °C. The DSC results showed that the Form C had a heat absorption peak of melting accompanied by decomposition at about 189 °C. The NMR results showed that the sample was consistent with a structure provided by a client. To sum up, the Form C was an anhydrous crystal form.

### 5.4 Crystal form Form D

The XRPD results showed that the Form D was a solid with poor crystallinity. The TGA results showed that the Form D lost 0.9% weight when being heated to 140 °C, and might decompose at 190 °C. The NMR results showed that, an integral at 6.48 ppm indicated that the free state:the fumaric acid in the sample was ≈1:1, and indicated that the sample was 1 fumarate; and an integral at 2.30 ppm indicated that there was small amount of toluene. To sum up, the Form D was suspected to be an anhydrous crystal form with a salt formation ratio (free state: fumaric acid) of 1:1.

**Table 7 Crystal form characterization result**

| Crystal form | TGA weight loss mass proportion % /Temperature range °C | DSC peak temperature °C | Solvation information |
|---|---|---|---|
| Form A | 0.0/25-150 | Heat absorption: 188.4 | anhydrate |
| Form B | 2.9/25-150 | Heat absorption: 57.2, 146.6, 178.4 | anhydrate |
| Form C | 0.0/RT-120 | 189.3 | Anhydrous crystal form |
| Form D | 0.9/RT-140 | / | Anhydrous crystal form |

### Embodiment 6 Solid-state conversion relationship of crystal form

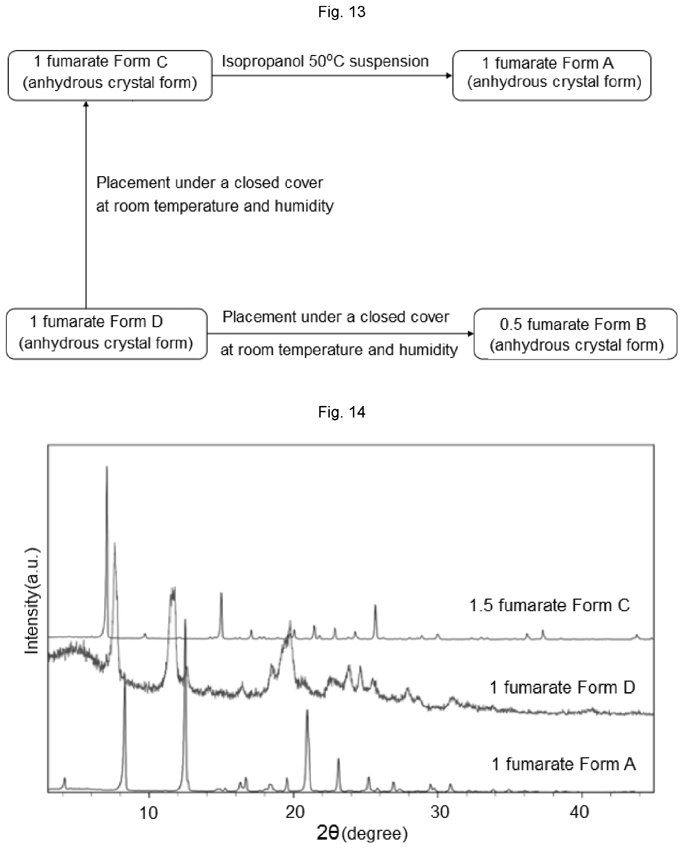

A conversion relationship of a solid state (crystal form/salt form) of the compound of the present disclosure was shown in Fig. 13. The Form C might be converted into the Form A through high-temperature suspension; and the Form D was converted into Form C+Form B after drying and placement under a closed cover at room temperature and humidity.

### Effect embodiment

### Embodiment 7 Stability

### 7.1. Method

About 20 mg of the sample was weighed and placed in a weighing bottle, and was separately placed in an open manner at high temperature (60 °C), high humidity (25 °C/92.5% RH), light (25 °C/4500 Lux), and acceleration (40 °C/75% RH), and sampling was performed for XRPD characterization and HPLC tests on Day 7 and Day 15.

### 7.2 Result

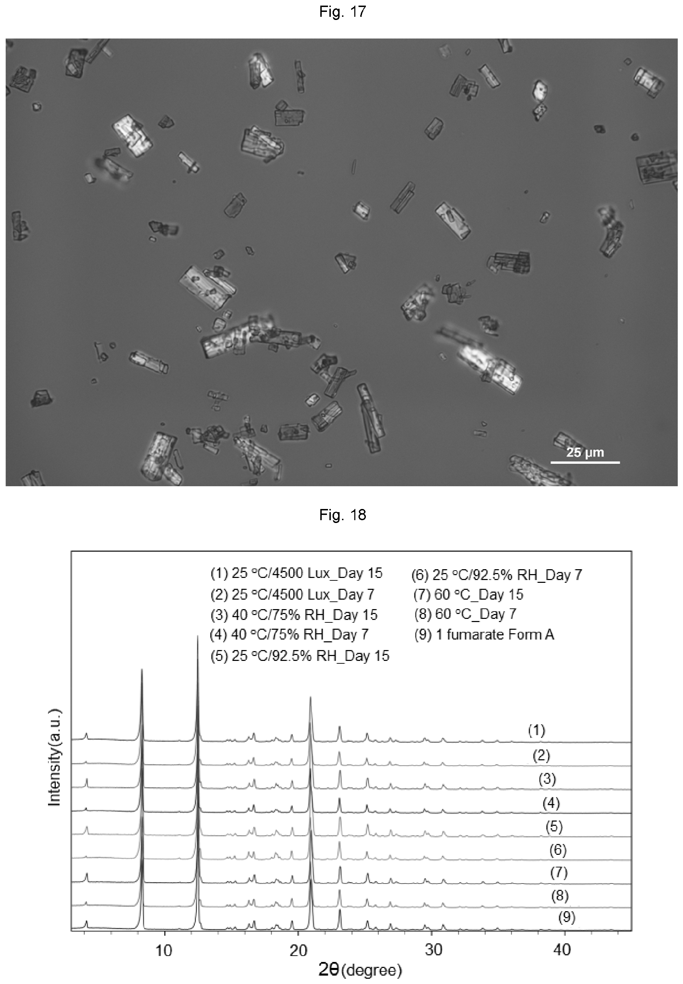

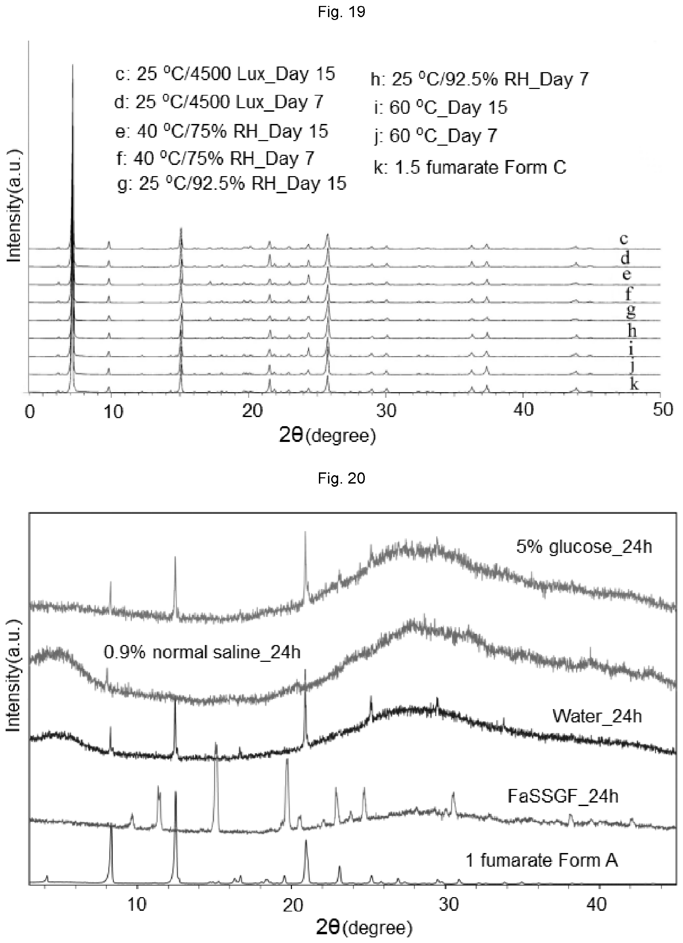

Results were shown in Table 8, Fig. 18, and Fig. 19. The XRPD results showed that, the XRPD on Day 0 was consistent with that on Day 15 when the Form A and the Form C were placed under conditions of high temperature, high humidity, acceleration, and light; the purity did not significantly change under conditions of high temperature, high humidity, and acceleration; and the purity reduced under light, and the solid turned yellow in appearance.

**Table 8 Stability study results**

| Crystal form | Condition | Day 7 | | Day 15 | |
|---|---|---|---|---|---|
| | | XRPD results | Purity/% | XRPD results | Purity/% |
| Form A Purity: 99.54% | High temperature 60 °C | No change in appearance and XRPD | 99.53 | No change in appearance and XRPD | 99.51 |
| | High humidity 25 °C/92.5% RH | No change in appearance and XRPD | 99.53 | No change in appearance and XRPD | 99.49 |
| | Acceleration 40 °C/75% RH | No change in appearance and XRPD | 99.49 | No change in appearance and XRPD | 99.50 |
| | Light 25 °C/4500 Lux | White powder turning yellow, no change in XRPD | 99.13 | White powder turning yellow, no change in XRPD | 98.76 |
| Form C Purity: 99.34% | High temperature 60 °C | No change in appearance and XRPD | 99.31 | No change in appearance and XRPD | 99.32 |
| | High humidity 25 °C/92.5% RH | No change in appearance and XRPD | 99.35 | No change in appearance and XRPD | 99.30 |
| | Acceleration 40 °C/75% RH | No change in appearance and XRPD | 99.36 | No change in appearance and XRPD | 99.33 |
| | Light 25 °C/4500 Lux | White powder turning yellow, no change in XRPD | 98.87 | White powder turning yellow, no change in XRPD | 98.32 |

### Embodiment 8 Solubility

### 8.1. Method

### 8.1.1 Solubility assessment in water

The sample was added in 4.0 mL water, vibrated at a constant temperature of 37 °C for 2 h, and then sampled; and the sampled solution was filtered by using a 0.22 µm water-based filter membrane, a diluent was used to properly dilute some samples with a high concentration, a signal peak area of the solution was measured with HPLC, and finally, a concentration of the compound in the solution was calculated according to the peak area, an HPLC standard curve of raw materials, and a dilution ratio. Furthermore, the XRPD test was performed on the remaining solid.

### 8.1.2 Solubility test

A preparation process of a biologic medium was shown in Table 9. The sample was added in the biologic medium, water, 0.9% normal saline, and 5% glucose, vibrated at a constant temperature of 37 °C for 24 h, and then sampled at 0.5 h, 2 h, and 24 h, respectively; and the sampled solutions were filtered by using a 0.22 µm water-based filter membrane, a diluent was used to properly dilute some samples with a high concentration, a signal peak area of the solution was measured with HPLC, and finally, a concentration of the compound in the solution was calculated according to the peak area, an HPLC standard curve of raw materials, and a dilution ratio. Furthermore, a pH value was tested by taking supernatant of 24 h, and the XRPD test was performed on the remaining solid.

**Table 9 Preparation process of biologic medium**

| Biologic medium | Preparation process |
|---|---|
| FaSSIF | 2.0843 g of an FaSSIF concentrated solution was weighed in a 50 mL volumetric flask and diluted with proper amount of pure water, then 111.7 mg of FaSSIF/FeSSIF/FaSSGF powder was added, and the mixture was made up to 50 mL and well mixed (pH being 6.5). |
| FeSSIF | 4.0705 g of an FeSSIF concentrated solution was weighed in a 50 mL volumetric flask and diluted with proper amount of pure water, then 559.7 mg of FaSSIF/FeSSIF/FaSSGF powder was added, and the mixture was made up to 50 mL and well mixed (pH being 5.0). |
| FaSSGF | 3.6754 g of an FaSSGF concentrated solution was weighed in a 100 mL volumetric flask and diluted with proper amount of pure water, then 6.0 mg of FaSSIF/FeSSIF/FaSSGF powder was added, and the mixture was made up to 100 mL and well mixed (pH being 1.6). |

### 8.2 Result

### 8.2.1 Form A

The results showed that, the solubility of the Form A in the biologic medium, water, normal saline, and glucose for 24 h was FaSSGF>water≈0.9% normal saline≈5% glucose>FeSSIF>FaSSIF from large to small. After 24 h of vibration in the FaSSIF, a very small amount of the solid remained; after 24 h of vibration in the FeSSIF, an oily substance appeared, after 24 h of vibration in the water, 0.9% normal saline, and 5% glucose, crystal forms of the remaining solids did not change, and the crystallinity deteriorated; and after 24 h of vibration in the FaSSGF, the XRPD of the remaining solids changed, and the NMR results showed that offset occurred at 6.40 ppm and 7.67 ppm, and peaks at 2.42 ppm and 6.47 ppm (fumaric acid) disappeared. Since there were small amounts of solids remained in the FaSSIF and the FaSSGF, a proper amount of the Form A was weighed and vibrated in FaSSIF and FaSSGF solutions for 24 h, and characterization was performed on the obtained solid. The XRPD results showed that, free fumaric acid was mixed in the remaining solid after 24 h of vibration in the FaSSGF, and ion chromatography test results showed that the content of chloride ions was 2.4%. The NMR results showed that, the ratio of the fumaric acid in the remaining solid after 24 h of vibration in the FaSSIF was reduced, and the ion chromatography test results showed that the content of chloride ions was 4.9%, and the content of phosphate ions was 2.9%. By synthesizing the above results, the Form A might dissociate in the FaSSIF and FaSSGF solutions, and part of the dissociated solid forms into a salt with an acidic component in the medium.

**Table 10 Dynamic solubility test**

| Crystal form | Medium | pH | Sample size (mg) | Volume (mL) | Solubility* (mg/mL) | | | Final pH | Remaining solid |
|---|---|---|---|---|---|---|---|---|---|
| | | | | | 0.5 h | 2 h | 24 h | | |
| Form A | FaSSIF | 6.5 | 20.2 | 4.0 | 3.64 | 2.87 | 1.99 | 5.0 | Dissociation |
| | FeSSIF | 5.0 | 39.8 | 4.0 | 4.29 | 2.34 | 2.88 | 4.8 | Oily substance |
| | FaSSGF | 1.7 | 40.1 | 4.0 | 6.17 | 6.37 | 3.78 | 2.1 | Dissociation |
| | Water | / | 20.1 | 4.0 | 3.09 | 3.08 | 3.17 | 3.7 | Form A |
| | 0.9% normal saline | / | 20.4 | 4.0 | 3.17 | 3.18 | 3.17 | 3.5 | Form A |
| | 5% glucose | / | 19.8 | 4.0 | 2.97 | 3.03 | 3.13 | 3.7 | Form A |

| | | | | | | | | | |
|---|---|---|---|---|---|---|---|---|---|
| Note: "*" represented a concentration of a corresponding free state in the solution calculated according to a free-state standard curve. | | | | | | | | | |

8.2.2 Form B The results showed that, the solubility of the Form B and the Form A in the water for 2 h was Form A>Form B from large to small; and after 2 h of vibration in the water, the crystal forms of the remaining solids did not change.

**Table 11 pH buffer solution and water solubility assessment results**

| Salt form | Medium | Sample size (mg) | Volume (mL) | Solubility (mg/mL)* | Final pH | Remaining solid XRPD |
|---|---|---|---|---|---|---|
| | | | | 2 h | | |
| Form B | Water | 20.1 | 4.0 | 1.57 | 4.7 | Form B |
| Form A | | 20.2 | 4.0 | 3.22 | 3.7 | Form A |

| | | | | | | |
|---|---|---|---|---|---|---|
| Note: "*" represented a concentration of a corresponding free state in the solution calculated according to a free-state standard curve. | | | | | | |

### 8.2.3 Form C

Solubility assessment was performed on the Form C in the water, an experimental method was shown in 8.1.1, and corresponding results were shown in Table 12.

**Table 12 Assessment results for solubility in water**

| Salt form | Medium | Sample (mg) | Volume (mL) | 2 h free-state solubility (mg/mL)^{a} | Final pH | Remaining solid XRPD |
|---|---|---|---|---|---|---|
| Form C | Water | 20.2 | 4.0 | 1.99 | 3.30 | Form C |

| | | | | | | |
|---|---|---|---|---|---|---|
| ^{a} was calculated according to an HPLC standard curve of a free state. | | | | | | |

### Embodiment 9 Hygroscopicity

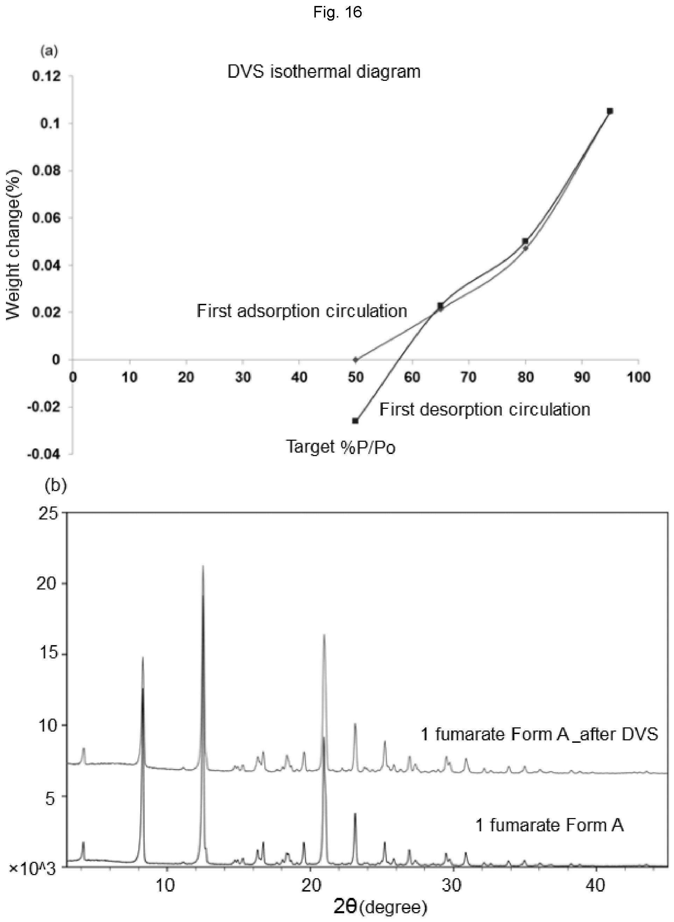

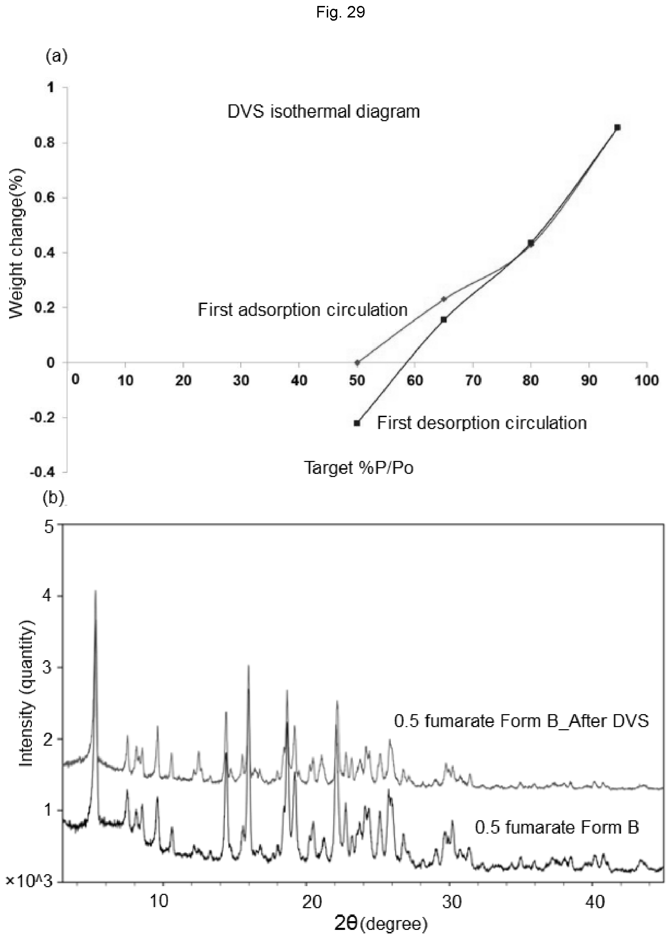

A DVS test was performed on the Form A and Form B, as shown in Fig. 16 and Fig. 29. The results showed that, the Form A had an adsorption weight gain of about 0.105% at 95% RH, an adsorption weight gain of about 0.047% at 80% RH, a desorption weight gain of about 0.050% at 80% RH, and a desorption weight loss of about 0.026% at 50% RH.

**Table 13 Summary table of preliminary assessment**

| Salt form | | Form A |
|---|---|---|
| 2 h free-state solubility (mg/mL) | Water | 3.22 |
| Solubility assessment for | Water | Form A |
| remaining solid XRPD | | |
| Hygroscopicity assessment | Adsorption weight gain at 80%RH (%) | 0.047 |
| | Adsorption weight gain at 95%RH (%) | 0.105 |
| | Desorption weight gain at 80%RH (%) | 0.026 |
| | XRPD results | Form A |

The Form B had an adsorption weight gain of about 0.855% at 95% RH, an adsorption weight gain of about 0.427% at 80% RH, a desorption weight gain of about 0.435% at 80% RH, and a desorption weight loss of about 0.221% at 50% RH. The XRPD of the Form A and the Form B did not significantly change after the DVS test.

**Table 14 Summary table of preliminary assessment**

| Salt form | | Form B |
|---|---|---|
| 2 h free-state solubility (mg/mL) | Water | 1.57 |
| Solubility assessment for remaining solid XRPD | Water | Form B |
| Hygroscopicity assessment | Adsorption weight gain at 80%RH (%) | 0.427 |
| | Adsorption weight gain at 95%RH (%) | 0.855 |
| | Desorption weight gain at 80%RH (%) | 0.435 |
| | XRPD results | Form B |

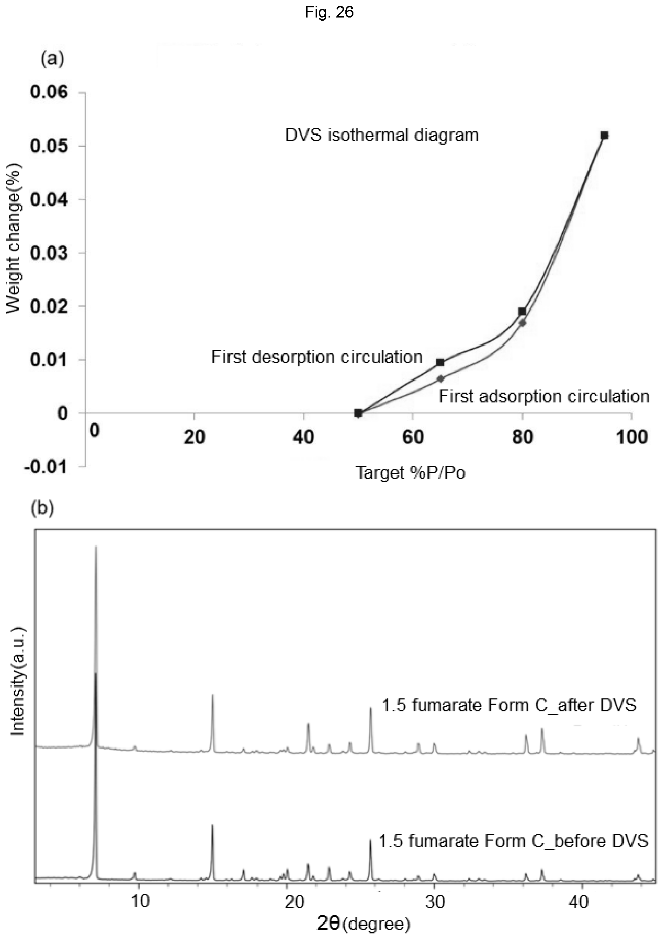

The DVS test was performed on the Form C, as shown in Fig. 26. The results showed that, the Form C had a weight gain of 0.052% at 95% humidity, a weight gain of 0.017% at 80% humidity during adsorption, and a weight gain of 0.019% at 80% humidity during desorption. The XRPD results indicated that the crystal form of the Form C did not change after the rapid DVS test.

**Table 15 Summary table of preliminary assessment**

| Salt form | | Form C |
|---|---|---|
| 2 h free-state solubility (mg/mL) | Water | 1.99 |
| Solubility assessment for remaining solid XRPD | Water | Form C |
| Hygroscopicity assessment | Adsorption weight gain at 80%RH (%) | 0.017 |
| | Adsorption weight gain at 95%RH (%) | 0.052 |
| | Desorption weight gain at 80%RH (%) | 0.019 |
| | XRPD results | Form C |

### Embodiment 10: Study of dissolution results of sample-prepared tablet

API tablets (conventional tableting) were prepared with the Form A and the Form C, with a specification of 10 mg (calculated based on free alkali); a dissolution rate of a sample was investigated in a phosphate buffer solution with pH=6.8, an acetate buffer solution with pH=4.5, a hydrochloric acid solution with pH=1.2, and purified water, respectively; and data comparison was shown in Table 16 and Table 17.

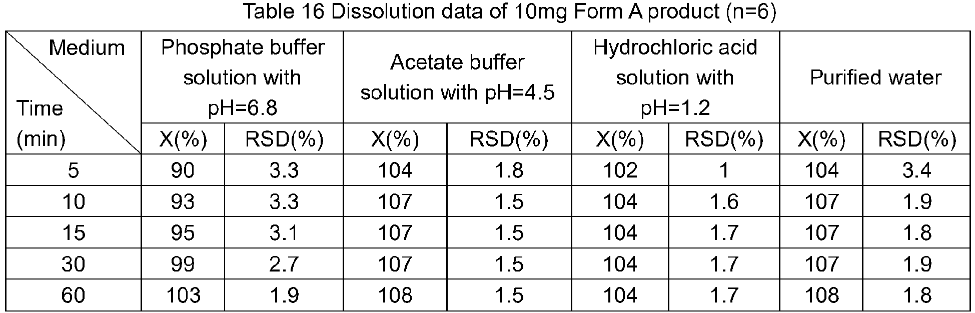

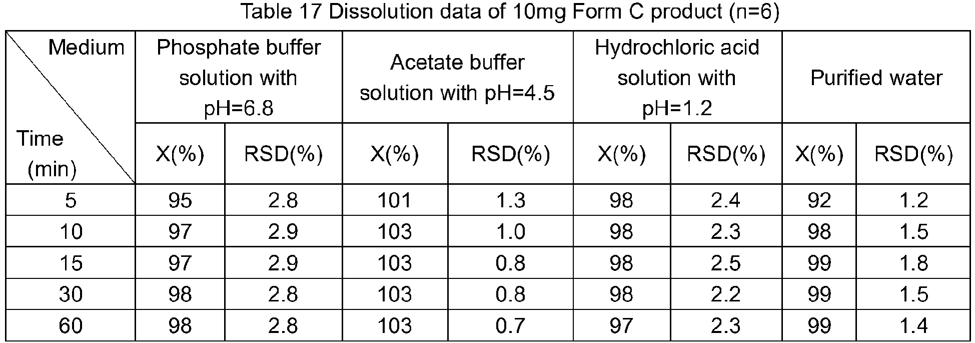

In conclusion, the dissolution results of the sample-prepared tablets indicated that, the dissolution rates of the tablets prepared with the Form A and the Form C in the phosphate buffer solution with pH=6.8, the acetate buffer solution with pH=4.5, the hydrochloric acid solution with pH=1.2, and the purified water within 15 min were all greater than 85%.

All literature mentioned in the present disclosure is cited by reference in the present disclosure as if each piece of the literature were cited separately as a reference. In addition, it should be understood that, after reading the above lecturing content in the present disclosure, those skilled in the art may make various alterations or modifications to the present disclosure, and these equivalent forms likewise fall within the scope defined by the claims appended to the present disclosure.

## Claims

1. A pyrrole sulfonamide acid inhibitor crystal, wherein a chemical structural formula of the crystal is shown in a Formula I: 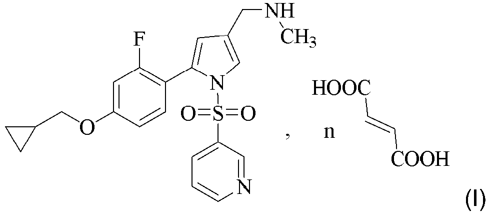 n=0.1-2.0.

2. The crystal according to claim 1, wherein the crystal is an anhydrate, and/or n is 0.5-1.5.

3. The crystal according to claim 1, wherein the crystal has a crystal form Form A, the crystal form Form A also has one or more features selected from the following groups:
(i1) an XRPD diagram of the crystal form Form A comprises 6 or more than 6 2θ values selected from the following groups: 4.1°±0.2°, 8.3°±0.2°, 12.5°±0.2°, 16.7°±0.2°, 19.6°±0.2°, 20.9°±0.2°, 23.1°±0.2°, 25.2°±0.2°, and 26.9°±0.2°;
(i2) the XRPD diagram of the crystal form Form A comprises 3 or more than 3 2θ values selected from the following groups: 4.1°±0.2°, 8.3°±0.2°, 12.5°±0.2°, 19.6°±0.2°, 20.9°±0.2°, 23.1°±0.2°;
(i3) the XRPD diagram of the crystal form Form A is **characterized in** Fig. 1;
(i4) the crystal form Form A loses no weight at 20-150 °C;
(i5) a TGA diagram of the crystal form Form A is **characterized in** Fig. 2;
(i6) a peak temperature of a DSC diagram of the crystal form Form A is 188.4 °C;
(i7) the DSC diagram of the crystal form Form A is **characterized in** Fig. 2;
(i8) the XRPD diagram of the crystal form Form A comprises 1 or more than 1 2θ values selected from the following groups: 4.1°±0.2°, 8.3°±0.2°, 12.5°±0.2°, 20.9°±0.2°, and 23.1°±0.2°; and
(i9) n of the crystal form Form A is =1.

4. The crystal according to claim 1, wherein the crystal has a crystal form Form B, the crystal form Form B also has one or more features selected from the following groups:
(j1) an XRPD diagram of the crystal form Form B comprises 6 or more than 6 2θ values selected from the following groups: 5.4°±0.2°, 7.5°±0.2°, 8.2°±0.2°, 8.5°±0.2°, 9.6°±0.2°, 14.5°±0.2°, 16.0°±0.2°, 18.7°±0.2°, 19.2°±0.2°, 22.1°±0.2°, 22.8°±0.2°, 24.1°±0.2°, and 25.9°±0.2°;
(j2) the XRPD diagram of the crystal form Form B comprises 3 or more than 3 2θ values selected from the following groups: 5.4°±0.2°, 14.5°±0.2°, 16.0°±0.2°, 18.7°±0.2°, and 22.1°±0.2°;
(j3) the XRPD diagram of the crystal form Form B is **characterized in** Fig. 4;
(j4) the crystal form Form B loses 2.9% weight at 20-150 °C;
(j5) a TGA diagram of the crystal form Form B is **characterized in** Fig. 5;
(j6) peak temperatures of a DSC diagram of the crystal form Form B are 57.2 °C, 146.6 °C, and 178.4 °C;
(j7) the DSC diagram of the crystal form Form B is **characterized in** Fig. 5;
(j8) the XRPD diagram of the crystal form Form B comprises 1 or more than 1 2θ values selected from the following groups: 5.3°±0.2°, 9.6°±0.2°, 14.4°±0.2°, 16.0°±0.2°, 18.7°±0.2°, and 22.1°±0.2°; and
(j9) n of the crystal form Form B is =0.5.

5. The crystal according to claim 1, wherein the crystal has a crystal form Form C, the crystal form Form C also has one or more features selected from the following groups:
(k1) an XRPD diagram of the crystal form Form C comprises 6 or more than 6 2θ values selected from the following groups: 7.1°±0.2°, 9.7°±0.2°, 15.0°±0.2°, 17.1°±0.2°, 20.1°±0.2°, 21.5°±0.2°, 22.9°±0.2°, 23.8°±0.2°, 25.7°±0.2°, 30.0°±0.2°, and 37.3°±0.2°;
(k2) the XRPD diagram of the crystal form Form C is **characterized in** Fig. 7;
(k3) the crystal form Form C loses no weight at 20-120 °C;
(k4) a TGA diagram of the crystal form Form C is **characterized in** Fig. 8;
(k5) a peak temperature of a DSC diagram of the crystal form Form C is 189.3 °C;
(k6) the DSC diagram of the crystal form Form C is **characterized in** Fig. 8;
(k7) the XRPD diagram of the crystal form Form C comprises 3 or more than 3 2θ values selected from the following groups: 7.1°±0.2°, 9.7°±0.2°, 15.0°±0.2°, 21.5°±0.2°, 22.9°±0.2°, and 25.7°±0.2°;
(k8) the XRPD diagram of the crystal form Form C comprises 1 or more than 1 2θ values selected from the following groups: 7.1°±0.2°, 9.7°±0.2°, 15.0°±0.2°, 21.5°±0.2°, 22.9°±0.2°, 25.7°±0.2°; and
(k9) n of the crystal form Form C is =1.5.

6. The crystal according to claim 1, wherein the crystal has a crystal form Form D, the crystal form Form D also has one or more features selected from the following groups:
(m1) an XRPD diagram of the crystal form Form D comprises 6 or more than 6 2θ values selected from the following groups: 7.6°±0.2°, 11.7°±0.2°, 12.6°±0.2°, 14.1°±0.2°, 16.5°±0.2°, 18.5°±0.2°, 19.8°±0.2°, 22.6°±0.2°, 23.9°±0.2°, 24.6°±0.2°, 25.5°±0.2°, 28.0°±0.2°, 28.6°±0.2°, and 31.1°±0.2°;
(m2) the XRPD diagram of the crystal form Form D is **characterized in** Fig. 10;
(m3) the crystal form Form D loses 0.9% weight at 20-140 °C;
(m4) a TGA diagram of the crystal form Form D is **characterized in** Fig. 11;
(m5) the XRPD diagram of the crystal form Form D comprises 3 or more than 3 2θ values selected from the following groups: 7.6°±0.2°, 11.7°±0.2°, 19.8°±0.2°, 23.9°±0.2°, and 24.6°±0.2°;
(m6) the XRPD diagram of the crystal form Form D comprises 1 or more than 1 2θ values selected from the following groups: 7.6°±0.2°, 11.7°±0.2°, and 19.8°±0.2°; and
(m7) n of the crystal form Form D is =1.

7. A method for preparing the crystal according to claim 1 or 3, wherein the crystal is of a crystal form Form A, and the method comprises the following steps:
(a1) taking a first substance, wherein the first substance is 1-[5-[4-(cyclopropylmethoxy)-2-fluorophenyl]-1-(pyridin-3-ylsulfonyl)-1H-pyrrole-3-yl]-N-methylmeth anamine; and
(a2) mixing the first substance with an organic solvent and fumaric acid for crystallization, so as to obtain the crystal form Form A, wherein
a molar ratio of the first substance to the fumaric acid is 1:0.5-1.5.

8. The preparation method according to claim 7, wherein the organic solvent is selected from any one of ethyl acetate, methyl acetate, butyl acetate, methanol, and ethanol or a combination thereof; and/or a mass-volume ratio of the first substance to the organic solvent is 1:5-30, preferably, 1:10-25, and more preferably 1:10-20.

9. A method for preparing the crystal according to claim 1 or 5, wherein the crystal is of a crystal form Form C, and the preparation method comprises any one of steps (c1) and (c2):
(c1) mixing a first substance with an organic solvent and fumaric acid to obtain the crystal form Form C,
wherein
the first substance is 1-[5-[4-(cyclopropylmethoxy)-2-fluorophenyl]-1-(pyridin-3-ylsulfonyl)-1H-pyrrole-3-yl]-N-methylmeth anamine, and a molar ratio of the first substance to the fumaric acid is 1:2.5-4.0; and
(c2) mixing a crystal form Form A with the organic solvent and the fumaric acid at 20-70 °C, so as to obtain the crystal form Form C.

10. The preparation method according to claim 9, wherein the organic solvent is selected from the following groups: ethyl acetate, methyl acetate, butyl acetate, methanol, and ethanol or a combination thereof; in step (c1), a mass ratio of the first substance to the fumaric acid is 3-0.5:1, preferably, 2-1:1; a mass-volume ratio of the first substance to the organic solvent is 1:5-30, preferably 1:10-25, and more preferably 1:10-20; and/or in step (c2), a molar ratio of the crystal form Form A to the fumaric acid is 1:1-1:5, preferably 1:1-1:2; and the organic solvent comprises methanol and/or ethanol.

11. A drug composition, conprising: (a) any one of the crystals shown in the Formula I in claims 1 to 6, and (b) a pharmaceutically acceptable ingredients or carrier.

12. An application of any one of the crystals shown in claims 1 to 6, wherein the crystal is used for preparing a Potassium Competitive Acid Blocker, and is used for preparing a drug or drug composition for treating erosive esophagitis, gastric ulcer, duodenal ulcer, indications for eradicating helicobacter pylori, and related diseases caused by excessive stomach acid.
